# EUROPÄISCHE PATENTANMELDUNG

(11) **EP 3 848 645 A1**
(43) Veröffentlichungstag der Anmeldung: **14.07.2021**
(21) Anmeldenummer: 21150827.0
(22) Anmeldetag: 11.01.2021
(51) Int. Cl.: F24F 8/30, A61L 9/22, A61L 9/20, F24F 11/30, F24F 8/192

(54) **ANORDNUNG FÜR EINE IONISIERUNGSEINRICHTUNG**

(30) Priorität: 13.01.2020 DE 102020200328; 18.12.2020 DE 102020216332
(71) Anmelder: Dauphin Entwicklungs- u. Beteiligungs GmbH, 91217 Hersbruck (DE)
(72) Erfinder: Heuer, André, 32683 Barntrup (DE); Schönhoff, Sven, 32816 Schieder-Schwalenberg (DE)
(74) Vertreter: Rau, Schneck & Hübner Patentanwälte Rechtsanwälte PartGmbB

(57) **Zusammenfassung**

Die Erfindung betrifft eine Einrichtungsanordnung, insbesondere für Gebäude, mit mindestens einem Tragelement (4) und mit mindestens einer an dem mindestens einen Tragelement (4) angeordneten Ionisierungseinrichtung (2), die mindestens ein Ionisierungselement (7) zur Ionisierung gasförmiger Medien, insbesondere von Luft, aufweist. Die Einrichtungsanordnung hat ferner eine mit der mindestens einen Ionisierungseinrichtung (2) zumindest zeitweise in Verbindung stehenden Betätigungseinrichtung (27) zum zumindest zeitweisen Betätigen der mindestens einen Ionisierungseinrichtung (2).

## Beschreibung

Der Inhalt der deutschen Patentanmeldung DE 10 2020 200 328.9 vom 13.01.2020 sowie der deutschen Patentanmeldung DE 10 2020 216 332.4 vom 18.12.2020 wird durch Bezugnahme hierin aufgenommen.

Die Erfindung betrifft eine Einrichtungsanordnung, insbesondere für Gebäude, wie Wohngebäude, Bürogebäude, Geschäfte oder dergleichen.

Oftmals ist in Gebäuden das Raumklima nicht zufriedenstellend. Dabei spielt die Qualität der Raumluft eine wichtige Rolle für die Gesundheit und das körperliche sowie geistige Wohlbefinden der dieser ausgesetzten Personen. Die Zusammensetzung der Raumluft ändert sich beispielsweise, wenn sich Personen, Pflanzen und/oder Tiere in einem Raum befinden. Häufig wird auch nicht richtig gelüftet, was zu einer schlechten Raumluft führt.

Die DE 25 09 767 B2 offenbart eine Vorrichtung zur Ionisierung von Luft in geschlossenen Räumen. Die Ionisierungsvorrichtung hat eine Austrittsöffnung für ionisierte Luft und ein Gehäuse, das als Untersatz für einen Fernseher ausgebildet ist. Innerhalb des Gehäuses befinden sich Seitenwände, die einen Luftkanal begrenzen. In diesem Luftkanal sind zwischen einer Ansaugöffnung und der Austrittsöffnung ein Gebläse mit zugehörigem Motor und ein Ionenerzeuger angeordnet.

Die DE 44 02 302 A1 offenbart einen Luftreiniger mit einer Ionisiereinrichtung, die in einem Gehäuse mit einem Einströmkanal und einem Ausströmkanal untergebracht ist. Dem Einströmkanal gegenüber gestellt ist eine Aromatisiereinrichtung, der ein Ventilator zur Luftdurchwälzung zugeordnet ist. Der Luftreiniger hat eine elektronische Regelung, die in Abhängigkeit gemessener Luftgütewerte die Ionisiereinrichtung steuert.

In der DE 31 09 528 A1 ist ein Raumluft-Ionisator offenbart. Der Raumluft-Ionisator umfasst ein in ein Filterteil und Ionisierungsteil unterteiltes Gehäuse. Das Filtergehäuseteil hat Lufteinlassöffnungen. Das Ionisierungs-Gehäuseteil weist eine Luftauslassöffnung auf, die mit einer Platte mit beliebig verstellbaren Kugeldüsen abgedeckt ist. In dem Filtergehäuseteil sitzt ein Lüfter.

Die Ionisierungswirkung des Stands der Technik ist oftmals nicht zufriedenstellend.

Der Erfindung liegt die Aufgabe zugrunde, eine Einrichtungsanordnung zu schaffen, die im Stande ist, ein besonders gutes Raumklima zu liefern.

Diese Aufgabe wird erfindungsgemäß durch die in dem unabhängigen Anspruch 1 angegebenen Merkmale gelöst. Der Kern der Erfindung liegt in einer Einrichtungsanordnung, die Teil einer Raumgestaltung bzw. -ausstattung ist und mindestens ein Ionisierungselement zur Ionisierung gasförmiger Medien, insbesondere Luft, aufweist.

Das mindestens eine Ionisierungselement ist beispielsweise elektrisch leitend und steht im Betrieb dann bevorzugt unter Hochspannung. Vorzugsweise ist das mindestens eine Ionisierungselement metallisch. Es ist zum Beispiel nadelartig, stiftartig, domartig oder dergleichen und erzeugt im Betrieb, insbesondere durch Koronaentladung und Feldemission, insbesondere negativ geladene, Ionen. Vorzugsweise sind die erzeugten Ionen nach maximal 60 s, bevorzugt maximal 30 s, bevorzugt maximal 15 s, bevorzugt maximal 10 s, rekombiniert. Das mindestens eine Ionisierungselement ist bevorzugt gegenüber dem mindestens einen Tragelement elektrisch isoliert. Das mindestens eine Tragelement ist gemäß einer bevorzugten Ausführungsform elektrisch nicht leitend. Alternativ ist das mindestens eine Ionisierungselement zum Beispiel imstande, durch Strahlung, wie Ultraviolettstrahlung, insbesondere negativ geladene, Ionen zu erzeugen. Es ist dann beispielsweise als entsprechende Lampe, Laser, Diode oder dergleichen ausgebildet. Die Ionen werden durch elektrische Ionisation von Atomen und/oder Molekülen erzeugt. Sie sind elektrisch geladen.

Es ist zweckmäßig, wenn die mindestens eine Ionisierungseinrichtung eine Vielzahl an Ionisierungselementen umfasst. Die Ionisierungselemente der mindestens einen Ionisierungseinrichtung sind beispielsweise kammartig angeordnet. Es ist von Vorteil, wenn die mindestens eine Ionisierungseinrichtung bzw. das mindestens eine Ionisierungselement beabstandet zu der Betätigungseinrichtung, insbesondere zur Verhinderung von Beeinträchtigungen der Ionen(erzeugung) angeordnet ist.

Die mindestens eine Ionisierungseinrichtung ist zum Beispiel im Stande, Luft zu reinigen. Dies ist vor allem auf eine staubbindende Wirkung zurückzuführen. Erzeugte negativ geladene Ionen lagern sich an Staub- bzw. Schmutzpartikeln in der Luft an, was eine Clusterbildung begünstigt. Derartige Cluster sind einfacher als Einzelpartikel filterbar. Sie setzen sich außerdem im Allgemeinen ab und können dann einfach entfernt, wie abgesaugt, werden. Gemäß einer bevorzugten Ausführungsform entsteht bei der Ionisierung hochreaktives Ozon, das auch zur Luftreinigung beiträgt. Es bewirkt eine Spaltung vieler geruchsbildender Moleküle. Zulässige Grenzwerte bzw. Konzentrationen für Ozon, beispielsweise der EU, werden eingehalten und, bevorzugt weit, unterschritten. Insgesamt ist durch die Ionisierung umgebender Luft das persönliche Wohlbefinden steigerbar.

Die mindestens eine Ionisierungseinrichtung bzw. das mindestens eine Ionisierungselement ist bevorzugt in einem zentralen Bereich an dem mindestens einen Tragelement angeordnet, insbesondere wenn das mindestens eine Tragelement als Deckenanordnung, Decke oder dergleichen ausgeführt ist. Eine zentrale Anordnung gewährleistet eine möglichst gute bzw. gleichmäßige Verteilung der Ionen. Alternativ ist diese/s in mindestens einem dezentralen Bereich dort angeordnet.

Es ist zweckmäßig, wenn die Positionierung der mindestens einen Ionisierungseinrichtung bzw. des mindestens einen Ionisierungselements derart ist, dass diese/s einen möglichst großen Abstand zu, insbesondere positiv, elektrisch aufgeladenen bzw. aufladbaren Komponenten der Einrichtungsanordnung, die zumindest teilweise aus Polyester und/oder Acrylglas bestehen, und/oder mindestens einer Einrichtung für die Datenverarbeitung, sofern vorhanden, aufweist. So ist eine Beeinflussung auf die Verteilung der, insbesondere negativen, Ionen wirksam reduzierbar bzw. vermeidbar.

Die Einrichtungsanordnung ist selbst nicht Teil der baulichen Struktur, insbesondere eines Gebäudes. Sie stützt sich gegenüber dieser ab. Beispielsweise ist die Einrichtungsanordnung mit der baulichen Struktur fest, bevorzugt aber wieder entfernbar, verbunden. Alternativ ist die Einrichtungsanordnung gegenüber der baulichen Struktur unfixiert und zum Beispiel gegenüber derselben verlagerbar, wie verschiebbar oder verfahrbar. Die Einrichtungsanordnung ist beispielsweise als Möbelstück, insbesondere Büro-Möbelstück, oder Raum-in-Raum-System bzw. -Anordnung, beispielsweise für Telefonate, Besprechungen und/oder Projekte, ausgebildet.

Das mindestens eine Tragelement ist vorzugsweise starr. Es ist beispielsweise ein Grundkörper, Gestell, Rahmen, Korpus oder Bestandteil desselben. Gemäß einer bevorzugten Ausführungsform ist das mindestens eine Tragelement plattenartig, leistenartig, fußartig oder dergleichen. Es ist zweckmäßig, wenn die mindestens eine Ionisierungseinrichtung mit dem mindestens einen Tragelement in direkter oder indirekter Verbindung steht.

Die Betätigungseinrichtung steht mit der mindestens einen Ionisierungseinrichtung zumindest zeitweise in direkter oder indirekter Verbindung, wie Signalverbindung bzw. elektrisch leitender Verbindung. Sie ist zum Beispiel, insbesondere direkt oder indirekt, manuell bzw. über Signale betätigbar. Beispielsweise ist die Betätigungseinrichtung durch mindestens einen Schalter, eine Betätigungstaste oder dergleichen betätigbar bzw. gebildet. Es ist zweckmäßig, wenn die Betätigungseinrichtung, insbesondere drahtlos, fernbedienbar ist. Günstigerweise sind vorhandene Bedienelemente nutzbar. Beispielsweise ist die Betätigungseinrichtung im Stande, das mindestens eine Ionisierungselement einzuschalten, auszuschalten und/oder im Betrieb zu halten.

Weitere vorteilhafte Ausgestaltungen der Erfindung sind in den Unteransprüchen angegeben.

Es ist zweckmäßig, wenn das mindestens eine Ionisierungselement zumindest zeitweise mit mindestens einer Energiequelle zur zumindest zeitweisen Versorgung mit elektrischer Energie in Verbindung steht. Die mindestens eine Energiequelle ist insbesondere im Stande, im Betrieb das mindestens eine Ionisierungselement mit elektrischer Spannung, wie Hochspannung, vorzugsweise zwischen 1.000 Volt und 5.000 Volt, vorzugsweise zwischen 1.000 Volt und 5.500 Volt, zu versorgen. Dabei fließende Ströme sind bevorzugt äußerst klein. Sie liegen beispielsweise zwischen 0,5 µA und 5 µA. Die mindestens eine Energiequelle steht beispielsweise mit der Betätigungseinrichtung zumindest zeitweise in elektrisch leitender Verbindung oder bildet mit dieser eine Einheit. Alternativ umfasst die Betätigungseinrichtung zum Beispiel die mindestens eine Energiequelle.

Eingangsgrößen der Betätigungseinrichtung gemäß dem Unteranspruch 2 sind beispielsweise manuelle Bediensignale und/oder Rückmeldesignale, wie von Sensoren. Das mindestens eine Ionisierungselement ist beispielsweise ansteuerbar und/oder regelbar, bevorzugt nur ansteuerbar.

Gemäß einer alternativen Ausführungsform kommt die Betätigungseinrichtung ohne Steuerung bzw. Regelung aus. Sie ist dann beispielsweise durch mindestens einen Schalter, eine Betätigungstaste oder dergleichen betätigbar bzw. gebildet.

Die mindestens eine Luftstrom-Anordnung gemäß dem Unteranspruch 3 hat günstigerweise eine Strömungs-Erzeugungsvorrichtung und ist beispielsweise als Belüftungsanordnung ausgebildet. Es ist zweckmäßig, wenn die mindestens eine Luftstrom-Anordnung im Stande ist, (Frisch-)Luft zuzuführen bzw. fördern und durch die mindestens eine Ionisierungseinrichtung erzeugte Ionen in Bewegung zu versetzen, wie zu verteilen. Es ist von Vorteil, wenn die mindestens eine Luftstrom-Anordnung im Stande ist, insbesondere gebrauchte, Luft abzuführen bzw. zu fördern. Bevorzugt ist der mindestens eine Luftstrom zumindest anfangs geführt.

Beispielsweise ist die mindestens eine Luftstrom-Anordnung imstande, mindestens einen Luftstrom durch Blasen bzw. Drücken zu erzeugen. Alternativ oder zusätzlich ist sie imstande, mindestens einen Luftstrom durch Saugen zu erzeugen. Die mindestens eine Luftstrom-Anordnung für den Saugbetrieb umfasst bevorzugt mindestens einen Radial-Ventilator.

Das mindestens eine Ionisierungselement ist in Strömungsrichtung des mindestens einen Luftstroms der mindestens einen Luftstrom-Anordnung nachgeordnet. Dies gilt insbesondere, wenn die mindestens eine Luftstrom-Anordnung den mindestens einen Luftstrom durch Blasen bzw. Drücken erzeugt. Vorzugsweise ist das mindestens eine Ionisierungselement in Strömungsrichtung des mindestens einen Luftstroms der mindestens einen Luftstrom-Anordnung alternativ vorgeordnet, insbesondere wenn die mindestens eine Luftstrom-Anordnung den mindestens einen Luftstrom durch Saugen erzeugt. Die räumlich beabstandete Anordnung des mindestens einen Ionisierungselements und der mindestens einen Luftstrom-Anordnung zueinander verhindert bzw. reduziert wirksam statische elektrische Aufladungen der mindestens einen Luftstrom-Anordnung.

Es ist zweckmäßig, wenn ein Abstand zwischen einem Einlass und/oder Auslass der mindestens einen Luftstrom-Anordnung und einem benachbarten Ionisierungselement vorliegt und bevorzugt mindestens 300 mm, bevorzugt mindestens 400 mm, bevorzugt mindestens 500 mm, bevorzugt mindestens 600 mm, beträgt. Günstigerweise beträgt ein Abstand zwischen einem Einlass und/oder Auslass der mindestens einen Luftstrom-Anordnung und einem benachbarten Ionisierungselement höchstens 1000 mm, bevorzugt höchstens 900 mm, bevorzugt höchstens 800 mm.

Wenn mehrere Ionisierungselemente bzw. Ionisierungseinrichtungen vorhanden sind, so erstrecken sich diese bevorzugt parallel zu der mindestens einen Luftstrom-Anordnung bzw. zu mindestens einen Einlass und/oder Auslass derselben.

Es ist zweckmäßig, wenn das mindestens eine Ionisierungselement in der mindestens einen Luftstrom-Anordnung angeordnet ist. Vorzugsweise ist das mindestens eine Ionisierungselement in bzw. an der mindestens einen Luftstrom-Anordnung angeordnet. Günstigerweise ist es an mindestens einer (Auslass)Lamelle der mindestens einen Luftstrom-Anordnung angeordnet. Alternativ oder zusätzlich ist es beispielsweise in einem Strömungskanal der mindestens einen Luftstrom-Anordnung angeordnet. Ionisierte Luft ist so besonders gut verteilbar und zu gewünschten Stellen förderbar. Ionen werden beispielsweise zusammen mit der Luft gefördert.

Auch die Anordnung gemäß dem Unteranspruch 4 führt zu einer besonders guten Verteilung der Ionen in der Luft, zum Beispiel in einem Raum. Das mindestens eine Ionisierungselement wird so beispielsweise entsprechend angeströmt bzw. umströmt. Ionen werden beispielsweise zusammen mit der Luft gefördert.

Die Ausgestaltung gemäß den Unteransprüchen 5, 6 ist besonders bediener- bzw. benutzerfreundlich. Beispielsweise wird die mindestens eine Ionisierungseinrichtung betätigt bzw. aktiviert, wenn die mindestens eine Luftstrom-Anordnung eingeschaltet und/oder in eine bestimmte Stufe gestellt wird. Alternativ wird zum Beispiel umgekehrt die mindestens eine Ionisierungseinrichtung deaktiviert, wenn die mindestens eine Luftstrom-Anordnung ausgeschaltet und/oder in eine bestimmte Stufe gestellt wird. Ein vorhandenes Bedienelement für die mindestens eine Luftstrom-Anordnung wird vorzugsweise genutzt.

Die Einrichtungsanordnung gemäß dem Unteranspruch 7 ist besonders einfach und funktionssicher. Die Ionisierungsleistung im Betrieb der mindestens einen Ionisierungseinrichtung ist bevorzugt unveränderbar und im Betrieb stets gleich. In den unterschiedlichen Stufen ist der Fördervolumenstrom der mindestens einen Luftstrom-Anordnung unterschiedlich.

Es ist zweckmäßig, wenn bei einer bevorzugten alternativen Ausführungsform bei einem erhöhten Fördervolumenstrom der mindestens einen Luftstrom-Anordnung die Betätigungseinrichtung die Leistung der mindestens einen Ionisierungseinrichtung entsprechend erhöht und/oder bei einem reduzierten Fördervolumenstrom die Betätigungseinrichtung die Leistung der mindestens einen Ionisierungseinrichtung entsprechend reduziert.

Die Ausgestaltung gemäß den Unteransprüchen 8 ist wieder äußerst bediener- bzw. benutzerfreundlich. Beispielsweise wird die mindestens eine Ionisierungseinrichtung betätigt bzw. aktiviert, wenn die mindestens eine Beleuchtungsanordnung eingeschaltet und/oder in eine bestimmte Beleuchtungsstufe gestellt wird. Alternativ wird zum Beispiel umgekehrt die mindestens eine Ionisierungseinrichtung deaktiviert, wenn die mindestens eine Beleuchtungsanordnung ausgeschaltet und/oder in eine bestimmte Beleuchtungsstufe gestellt wird. Ein vorhandenes Bedienelement für die mindestens eine Beleuchtungsanordnung wird vorzugsweise genutzt. Alternativ sind die mindestens eine Beleuchtungsanordnung und die mindestens eine Ionisierungseinrichtung unabhängig voneinander, wie unabhängig voneinander betätigbar bzw. ansteuerbar. Sie sind dann voneinander funktional getrennt.

Es ist zweckmäßig, wenn die mindestens eine Ionisierungseinrichtung bzw. das mindestens eine Ionisierungselement zwischen Beleuchtungsanordnungen, sofern mehrere vorhanden, angeordnet ist. Bevorzugt ist ein Abstand zwischen einer Ionisierungseinrichtung bzw. einem Ionisierungselement und einer Beleuchtungsanordnung, insbesondere zur Vermeidung von Beeinträchtigungen der Ionen(erzeugung), vorhanden und beträgt bevorzugt mindestens 100 mm, insbesondere mindestens 150 mm, bevorzugt mindestens 200 mm, bevorzugt mindestens 230 mm. Es ist zweckmäßig, wenn der Abstand zwischen 230 mm und 600 mm liegt. Bevorzugt liegt der Abstand bei einem Raummodul für Telefonate bzw. einem Minibüro, das vorzugsweise eine Grundfläche zwischen 0,7 qm und 2 qm aufweist, zwischen 230 mm und 300 mm. Bei einem Raummodul für Besprechungen, Projekte oder dergleichen liegt der Abstand vorzugsweise zwischen 260 mm und 600 mm, bevorzugt zwischen 300 mm und 500 mm.

Die mindestens eine Luftstrom-Anordnung verteilt bevorzugt eine an dem mindestens einen Ionisierungselement im Betrieb anliegende Hochspannung bzw. die erzeugten Ionen im Raum. Die Hochspannung sucht quasi insbesondere ein Nullpotential bzw. einen Massepunkt. Bei einer nicht dimmbaren Beleuchtungsanordnung, wie Leuchtdiodenanordnung, bleibt die Funktion einer Ionisierungsplatine und/oder einer Ionisierungsanzeige, beispielsweise gebildet durch mindestens eine Leuchtdiode, durch die Hochspannung unbeeinflusst. Ist die Beleuchtungsanordnung dagegen dimmbar, so erfolgt vorzugsweise eine Erdung eines Gehäuses der Beleuchtungsanordnung. Durch die Erdung des Gehäuses der Beleuchtungsanordnung sind die Ionisierungsplatine und die Ionisierungsanzeige geschützt.

Die Ausbildung gemäß dem Unteranspruch 9 ist äußerst effizient und benutzerfreundlich. Zum Beispiel ist die Leistung der mindestens einen Ionisierungseinrichtung bei keiner erfassten Person niedriger als bei mindestens einer erfassten Person. Beispielsweise wird die Leistung der mindestens einen Ionisierungseinrichtung reduziert bzw. die mindestens eine Ionisierungseinrichtung ausgeschaltet, wenn die mindestens eine Personen-Erfassungseinrichtung keine Person erfasst. Die Leistung der mindestens einen Ionisierungseinrichtung wird zum Beispiel erhöht bzw. wird die mindestens eine Ionisierungseinrichtung eingeschaltet, wenn die mindestens eine Personen-Erfassungseinrichtung mindestens eine Person erfasst. Die mindestens eine Ionisierungseinrichtung ist dann in Betrieb. Die mindestens eine Personen-Erfassungseinrichtung ist beispielsweise als Präsenz- und/oder Bewegungs-Meldeeinrichtung ausgeführt. Sie ist beispielsweise an mindestens einem Tragelement, wie einem Paneel, einer Deckenordnung, einem Boden oder dergleichen, zum Beispiel zentral oder dezentral, angeordnet. Eine umgekehrte Betätigung ist alternativ möglich. Gemäß einer alternativen, einfachen Ausgestaltung ist keine Personen-Erfassungseinrichtung vorhanden.

Es ist zweckmäßig, wenn die mindestens eine Personen-Erfassungseinrichtung imstande ist, Licht, sofern vorhanden, Belüftung, sofern vorhanden, und Ionisierung einzuschalten. Vorzugsweise steuert die mindestens eine Personen-Erfassungseinrichtung die Ionisierung automatisch mit einer Nachlaufzeit von mindestens einer, bevorzugt mindestens drei, bevorzugt mindestens fünf, Minuten an. Günstigerweise steuert die mindestens eine Personen-Erfassungseinrichtung die Ionisierung automatisch mit einer Nachlaufzeit von höchstens 30 Minuten, bevorzugt höchstens 20 Minuten, bevorzugt höchstens 10 Minuten, an. Wird die Ionisierung nachgerüstet oder ab Werk verbaut, läuft die Ionisierung bevorzugt nur parallel mit Licht, sofern vorhanden, und Luft, sofern vorhanden, im einfachen An/Aus-Betrieb.

Die Einrichtungsanordnung gemäß dem Unteranspruch 10 umfasst vorzugsweise ein (Arbeits-)Raummodul und die mindestens eine Ionisierungseinrichtung sowie die Betätigungseinrichtung. Das mindestens eine Tragelement ist bevorzugt durch eine Deckenanordnung bzw. ein Deckenpaneel oder eine Seitenwand des Raummoduls gebildet. Es ist zweckmäßig, wenn alternativ mindestens eine Seitenwand als Akustikelement ausgebildet und vorzugsweise mit einem Stoff, bevorzugt einem Polyesterstoff bzw. Wollstoff, bespannt ist. Bevorzugt besteht mindestens eine Seitenwand aus Glas. Es ist zweckmäßig, wenn bei dem Raummodul sämtliche Seitenwände aus Glas bestehen, sodass dieses quasi ringsum verglast ist. Es ist von Vorteil, wenn das mindestens eine Raummodul mindestens eine Tür, günstigerweise aus Glas, aufweist. Günstigerweise befindet sich die mindestens eine Luftstrom-Anordnung in einem zentralen Bereich einer Deckenanordnung, da sich dort darunter erfahrungsgemäß meistens die Nutzer aufhalten.

Es ist zweckmäßig, wenn die Anordnung der mindestens einen Ionisierungseinrichtung bzw. des mindestens einen Ionisierungselements derart ist, dass bei unterschiedlichen Raumgrößen dennoch eine gleichmäßige Verteilung der erzeugten Ionen erfolgt.

Günstigerweise umfasst die Deckenanordnung eine abgehängte Decke, die bevorzugt aus Blech, insbesondere aus Stahlblech, und/oder aus mindestens einer Spanplatte, insbesondere einer melaminbeschichteten Spanplatte, besteht.

Die Einrichtungsanordnung gemäß dem Unteranspruch 11 weist dagegen ein Möbelstück und die mindestens eine Ionisierungseinrichtung sowie die Betätigungseinrichtung auf. Das Möbelstück steht beispielsweise auf einem Untergrund, wie Boden. Es kann sich gegenüber einer Wand, insbesondere des Gebäudes, abstützen oder freistehend anordenbar sein. Günstigerweise erstreckt sich das mindestens eine Ionisierungselement horizontal. Eine vertikale Erstreckung ist alternativ möglich. Es ist zweckmäßig, wenn mehrere Ionisierungselemente vorhanden sind. Diese erstrecken sich bevorzugt in unterschiedliche Richtungen, insbesondere von mindestens einem Tragelement aus.

Das mindestens eine Gehäuseteil gemäß dem Unteranspruch 12 hat vorzugsweise einen länglichen, insbesondere zylindrischen, Grundkörper, der einen Aufnahmeraum zur Aufnahme des mindestens einen Ionisierungselements räumlich begrenzt. Bevorzugt hat das Gehäuseteil eine Länge zwischen 30 mm und 120 mm, vorzugsweise zwischen 45 mm und 85 mm. Bevorzugt weist das Gehäuseteil eine Querabmessung zwischen 15 mm und 60 mm, vorzugsweise zwischen 20 mm und 40 mm, auf. Es ist zweckmäßig, wenn jeweils ein Ionisierungselement in genau einem Gehäuseteil untergebracht ist.

Das Gehäuseteil gemäß dem Unteranspruch 13 hat eine besonders hohe Beständigkeit gegenüber Ozon, das sich im Betrieb gemäß einer bevorzugten Ausführungsform bildet. Ferner bleibt so die Verteilung erzeugter Ionen, insbesondere negativ geladener Ionen, unbeeinflusst. Vorzugsweise ist der Grundkörper vollständig aus dem Polymermaterial, insbesondere thermoplastischem Polymermaterial, insbesondere PVC (Polyvinylchlorid), gebildet.

Es ist zweckmäßig, wenn der mindestens eine Kopf gemäß dem Unteranspruch 14 gegenüber einem zugeordneten Grundkörper nach seitlich außen vorspringt. Günstigerweise ist der Kopf kegel- bzw. kegelstumpfförmig. Er ist in montiertem Zustand bevorzugt in der zu ionisierenden Umgebung bzw. benachbart zu dieser angeordnet. Es ist zweckmäßig, wenn im Längsschnitt eine Mantelfläche des Kopfs einen Winkel mit einer Deckfläche des Kopfs einschließt, der zwischen 35° und 55°, bevorzugt zwischen 40° und 50°, liegt. Bevorzugt beträgt der Winkel 45°.

Günstigerweise ist in dem mindestens einen Kopf in einem zentralen Bereich mindestens eine Ionisierungsöffnung ausgebildet, die eine Verbindung zwischen dem Aufnahmeraum bzw. dem mindestens einen Ionisierungselement und der Umgebung, wie einem Innenraum, herstellt. Luft, insbesondere geförderte Luft, ist so imstande, an das mindestens eine Ionisierungselement zu gelangen. Ferner sind die erzeugten Ionen imstande, in die Umgebung zu gelangen. Die Ausbildung als Kegel oder Kegelstumpf erlaubt eine besonders gute Verteilung negativ geladener Ionen. Vorzugsweise hat die Ionisierungsöffnung eine Querabmessung, insbesondere einen Durchmesser, der zwischen 5 mm und 15 mm, liegt.

Günstigerweise hat das mindestens eine Gehäuseteil mindestens einen, insbesondere separaten, Fuß. Der mindestens eine Fuß ist, vorzugsweise vollständig, aus einem Polymermaterial, insbesondere thermoplastischen Polymermaterial, insbesondere PVC, gebildet. Es ist zweckmäßig, wenn der mindestens eine Fuß als Kappe ausgeführt ist und mindestens ein Leuchtmittel, insbesondere eine Leuchtdiode, zum Anzeigen einer aktiven Ionisierung hat/trägt. Er ist bevorzugt mit einem Grundkörper, insbesondere lösbar, verbindbar.

Es ist zweckmäßig, wenn der mindestens eine Ferritkörper gemäß dem Unteranspruch 15 benachbart zu einem Anschlussbereich angeordnet ist, wo sich eine Anschlussleitung an das Schutzgehäuse anschließt. Bevorzugt ist der mindestens eine Ferritkörper zwischen dem Anschlussbereich und der mindestens einen Ionisierungsplatine angeordnet. Das Schutzgehäuse schützt bevorzugt vor Hochspannung und sichert so die mindestens eine Ionisierungsplatine. Der mindestens eine Ferritkörper sichert günstigerweise eine gleichmäßige Erzeugung negativ geladener Ionen und schützt vorzugsweise vor elektromagnetischen Feldern. Insbesondere schützt er vor induktiven und/oder kapazitiven Störfrequenzen.

Der mindestens eine Ferritkörper gemäß dem Unteranspruch 16 ist bevorzugt ringförmig. Günstigerweise ist/sind der Masseleiter, Nullleiter und/die Phase einer Anschlussleitung um den mindestens einen Ferritkörper unter Durchsetzung der mindestens einen Durchführung, bevorzugt unter Bildung einer Spule, Windung oder dergleichen, gewickelt. Die Windungen sind bevorzugt isoliert voneinander. Günstigerweise sind sie nebeneinander angeordnet und überdecken einander nicht.

Die mindestens eine Ionisierungseinrichtung ist bevorzugt nachrüstbar, also nachträglich einbaubar. Günstigerweise ist dies von einem Laien bewerkstelligbar. Die mindestens eine Ionisierungseinrichtung weist dafür vorzugsweise einen Stecker, wie einen "Plug and Play"-Anschluss, auf. Der Stecker ist beispielsweise in die Betätigungseinrichtung bzw. einen mit dieser in Verbindung stehenden Transformator steckbar.

Es ist zweckmäßig, wenn die mindestens eine Ionisierungseinrichtung eine Betriebszustandsanzeige aufweist und/oder mit einer solchen in Verbindung steht. Es ist so einfach erkennbar, ob diese aktuell in Betrieb ist. Es ist von Vorteil, wenn auch erkennbar ist, in welcher Stufe die mindestens eine Ionisierungseinrichtung aktuell läuft.

Es ist von Vorteil, wenn die Einrichtungsanordnung mindestens einen (weiteren) Sensor, wie Gassensor, insbesondere Kohlendioxidsensor, Kohlenmonoxidsensor und/oder Sensor für flüchtige organische Verbindungen (VOC), umfasst. Der mindestens eine (weitere) Sensor steht vorzugsweise zumindest zeitweise mit der Betätigungseinrichtung in Signalverbindung bzw. elektrisch leitender Verbindung. Bevorzugt betätigt die Betätigungseinrichtung die mindestens eine Ionisierungseinrichtung in Abhängigkeit momentan erfasster Anteile, Konzentrationen oder dergleichen, insbesondere der umgebenden Luft.

Nachfolgend werden unter Bezugnahme auf die beigefügte Zeichnung bevorzugte Ausführungsformen der Erfindung beispielhaft beschrieben. Dabei zeigen:
- Fig. 1: einen Vertikalschnitt durch eine erfindungsgemäße Einrichtungsanordnung,
- Fig. 2: eine Ansicht, die die in Figur 1 dargestellte Einrichtungsanordnung deckenseitig zeigt,

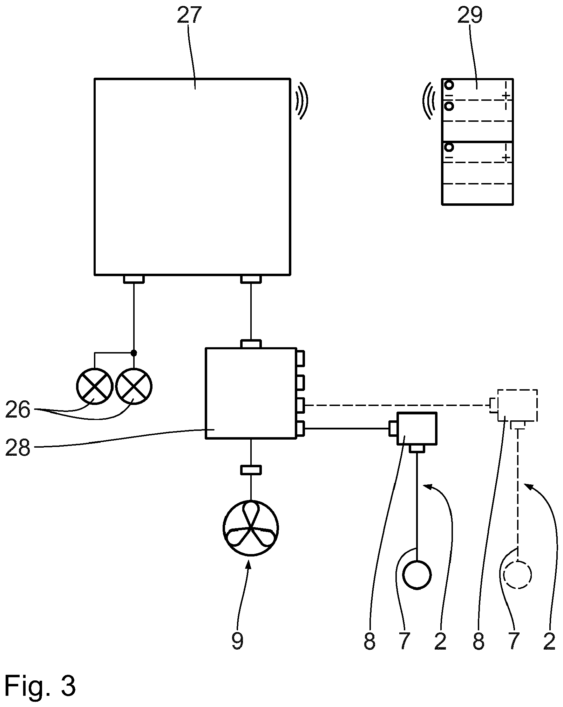

- Fig. 3: einen vereinfachten Schaltplan der im Allgemeinen in Figur 1 veranschaulichten Einrichtungsanordnung,
- Fig. 4: einen Horizontalschnitt durch eine erfindungsgemäße Einrichtungsanordnung gemäß einer zweiten Ausführungsform,
- Fig. 5: eine Vorderseite der in Fig. 4 gezeigten Einrichtungsanordnung,
- Fig. 6: eine Rückseite der in Fig. 4 veranschaulichten Einrichtungsanordnung,
- Fig. 7: eine Ansicht, die eine alternative Deckenanordnung einer erfindungsgemäßen Einrichtungsanordnung gemäß einer dritten Ausführungsform zeigt,
- Fig. 8: eine Deckenanordnung einer erfindungsgemäßen Einrichtungsanordnung gemäß einer vierten Ausführungsform,
- Fig. 9: einen Vertikalschnitt durch die Einrichtungsanordnung gemäß der in Fig. 8 veranschaulichten vierten Ausführungsform,
- Fig. 10: eine Deckenanordnung einer erfindungsgemäßen Einrichtungsanordnung gemäß einer fünften Ausführungsform,
- Fig. 11: einen Vertikalschnitt durch die in Fig. 10 gezeigte Einrichtungsanordnung gemäß der fünften Ausführungsform,
- Fig. 12: eine Deckenanordnung einer erfindungsgemäßen Einrichtungsanordnung gemäß einer sechsten Ausführungsform,
- Fig. 13: einen Vertikalschnitt durch die Einrichtungsanordnung gemäß der in Fig. 12 dargestellten sechsten Ausführungsform,
- Fig. 14: eine Deckenanordnung einer erfindungsgemäßen Einrichtungsanordnung gemäß einer siebten Ausführungsform,
- Fig. 15: einen Vertikalschnitt durch die Einrichtungsanordnung gemäß der in Fig. 14 veranschaulichten siebten Ausführungsform,
- Fig. 16a: einen vereinfachten alternativen Schaltplan für eine erfindungsgemäße Einrichtungsanordnung,
- Fig. 16b: einen vereinfachten alternativen Schaltplan für eine erfindungsgemäße Einrichtungsanordnung,
- Fig. 17: eine Seitenansicht eines Gehäuseteils für ein Ionisierungselement einer erfindungsgemäßen Einrichtungsanordnung,
- Fig. 18: eine Draufsicht auf das in Fig. 17 veranschaulichte Gehäuseteil,
- Fig. 19: eine Seitenansicht des in den Fig. 17, 18 dargestellten Gehäuseteils samt Ionisierungselement,
- Fig. 20: eine Ansicht, die Basiskomponenten einer Ionisierungseinrichtung einer erfindungsgemäßen Einrichtungsanordnung zeigt,
- Fig. 21: eine Draufsicht auf einen Ferritkörper der in Fig. 20 veranschaulichten Ionisierungseinrichtung,
- Fig. 22: eine Seitenansicht einer erfindungsgemäßen Einrichtungsanordnung gemäß einer weiteren Ausführungsform,
- Fig. 23: eine Draufsicht auf die in Fig. 22 dargestellte Einrichtungsanordnung, und
- Fig. 24: einen vereinfachten Schaltplan für die Einrichtungsanordnung gemäß Fig. 22, 23

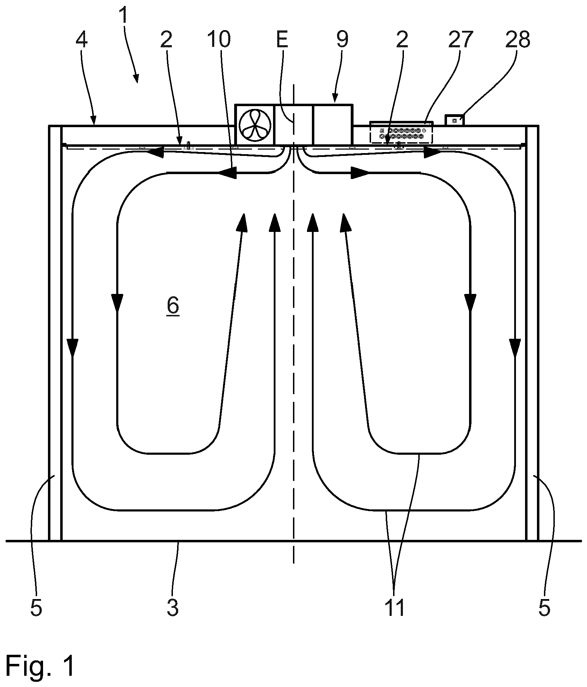

Bei einem in Figur 1 in der Gesamtheit dargestellten quaderförmigen Raummodul 1 sind deckenseitig zwei Ionisierungseinrichtungen 2 angeordnet. Das Raummodul 1 steht auf einem Boden 3 eines Gebäudes (nicht dargestellt) und bildet eine Einrichtungsanordnung. Die Anzahl der Ionisierungseinrichtungen 2 ist abhängig von der Größe des Raummoduls 1 und/oder einer gewünschten Intensität der Ionisierung, also Anzahl negativ geladener Ionen pro Kubikzentimeter.

Das Raummodul 1 hat eine Deckenanordnung 4, die in montiertem Zustand des Raummoduls 1 horizontal über dem Boden 3 verläuft. Ferner umfasst das Raummodul 1 Seitenwände 5, die sich jeweils randseitig an die Deckenanordnung 4 anschließen und plattenartig sind. Die Seitenwände 5 verlaufen jeweils senkrecht zu der Deckenanordnung 4 und in montiertem Zustand des Raummoduls 1 vertikal. Sie haben jeweils eine vertikale Höhe, die bevorzugt zwischen 2,20 m und 2,50 m liegt. Durch die Seitenwände 5 und die Deckenanordnung 4 ist ein Innenraum 6 des Raummoduls 1 nach außen räumlich begrenzt.

Jede Ionisierungseinrichtung 2 ist an der Deckenanordnung 4 angeordnet. Die Ionisierungseinrichtungen 2 haben einen identischen Abstand zu einer vertikalen Mittelebene E des Raummoduls 1. Sie liegen außerdem vorzugsweise in einer gemeinsamen weiteren Hauptebene H des Raummoduls 1, die senkrecht zu der Mittelebene E verläuft. Die Ionisierungseinrichtungen 2 sind identisch ausgebildet.

Jede Ionisierungseinrichtung 2 hat eine in den Innenraum 6 ragende, freie Ionisierungsnadel 7 und eine Schutzbox 8, die in die Deckenanordnung 4 eingesetzt bzw. an dieser angeordnet ist. In jeder Schutzbox 8 befindet sich eine Platine, die Bauteile und Verbindungen für den Betrieb der zugehörigen Ionisierungsnadel 7 trägt.

In die Deckenanordnung 4 ist außerdem zentral eine Belüftungsanordnung 9 zur Belüftung des Raummoduls 1 eingesetzt. Die Belüftungsanordnung 9 ist länglich und erstreckt sich parallel zu Seitenwänden 5 in der Mittelebene E. Sie ist mittig zwischen den Ionisierungseinrichtungen 2 und beabstandet zu diesen angeordnet.

Im Betrieb drückt die Belüftungsanordnung 9 Zuluft bzw. Frischluft von außen in den Innenraum 6 und erlaubt es, Abluft bzw. verbrauchte Luft durch diese wieder aus dem Innenraum 6 auszutreten. Dies ist in Figur 1 anhand der Strömungspfeile veranschaulicht. Die Strömungspfeile 10 zeigen die Strömung der Zuluft in dem Raummodul 1, während die Strömungspfeile 11 die Strömung der Abluft in dem Raummodul 1 veranschaulichen. In dem Raummodul 1 zeigt die strömende Luft günstigerweise einen Coanda-Effekt. Eine Ausgestaltung bzw. Luft-Strömung ohne Coanda-Effekt ist alternativ möglich.

Die Belüftungsanordnung 9 hat ein, insbesondere kastenartiges, Gehäuse 12, das in einer ersten Querwand 13 einen Zulufteinlass 14 aufweist. Der Zulufteinlass 14 umfasst ein Lamellengitter mit Zulufteinlassschlitzen.

Stromabwärts zu dem Zulufteinlass 14 hat die Belüftungsanordnung 9 eine Zuluftkammer 15, die sich in einer Zuluft-Hauptströmungssrichtung 16 verengt bzw. verjüngt.

Stromabwärts zu der Zuluftkammer 15 in Bezug auf die Zuluft-Hauptströmungsrichtung 16 weist die Belüftungsanordnung 9 einen ersten Schalldämpfer 17 auf, der sich in der Zuluft-Hauptströmungsrichtung 16 erstreckt und ein umfangsseitig mikroperforiertes, inneres Zuluftrohr und einen äußeren Dämmmaterialmantel umfasst.

Stromabwärts zu dem ersten Schalldämpfer 17 in Bezug auf die Zuluft-Hauptströmungsrichtung 16 weist die Belüftungsanordnung 9 ein Gebläse 18 auf, dessen Lüfterrad mittels eines Antriebsmotors drehantreibbar ist.

Stromabwärts zu dem Gebläse 18 in Bezug auf die Zuluft-Hauptströmungsrichtung 16 ist ein zweiter Schalldämpfer 19 angeordnet, der in seinem Aufbau und seiner Dimensionierung dem ersten Schalldämpfer 17 entspricht. Die Schalldämpfer 17, 19 sind so in Reihe geschaltet, wobei das Gebläse 18 zwischen diesen angeordnet ist.

Stromabwärts zu dem zweiten Schalldämpfer 19 in Bezug auf die Zuluft-Hauptströmungsrichtung 16 weist die Belüftungsanordnung 9 ein Strömungs-Umkehrelement 20 auf, das als Halbkreisbogen ausgebildet ist.

Stromabwärts zu dem Strömungs-Umkehrelement 20 in Bezug auf die Zuluft-Hauptströmungsrichtung 16 hat die Belüftungsanordnung 9 einen länglichen Zuluftauslass 21, der sich längs der dort vorherrschenden Zuluft-Hauptströmungsrichtung 16 erstreckt und parallel zu den Schalldämpfern 17, 19 verläuft. Der Zuluftauslass 21 weist ein Zuluftauslassgitter 22 mit Zuluftauslasslamellen auf, die sich längs der dort vorherrschenden Zuluft-Hauptströmungsrichtung 16 unter Bildung von Zuluftauslassschlitzen erstrecken. Das Zuluftauslassgitter 22 ist derart ausgebildet und angeordnet, dass die Zuluft in dem Innenraum 6 des Raummoduls 1 in einander gegensinnigen Richtungen nach seitlich außen geführt wird.

Der Zulufteinlass 14, die Zuluftkammer 15, die Schalldämpfer 17, 19, das Gebläse 18, das Strömungs-Umkehrelement 20 und der Zuluftauslass 21 bilden eine Zulufteinrichtung für Zuluft.

Eine Ablufteinrichtung der Belüftungsanordnung 9 ist seitlich neben der Zulufteinrichtung angeordnet. Die Ablufteinrichtung hat benachbart zu der ersten Querwand 13 und einer gegenüberliegenden zweiten Querwand 23 jeweils einen Ablufteinlass 24 mit einer Vielzahl von Ablufteinlassschlitzen. Zwischen den Ablufteinlässen 24 weist die Ablufteinrichtung in einer äußeren benachbarten Längswand der Belüftungsanordnung 9 einen gemeinsamen zentralen Abluftauslass auf.

Die Ablufteinrichtung hat ferner zwischen jedem Ablufteinlass 24 und dem Abluftauslass zwei Abluft-Umlenkkörper 25, die jeweils im Querschnitt im Wesentlichen dreieckförmig und an gegenüberliegenden Seiten der Ablufteinrichtung angeordnet sind.

Im Betrieb saugt das Gebläse 18 über seine Saugseite Zuluft aus der Umgebung, also von außerhalb des Innenraums 6, ein. Diese Zuluft durchtritt den Zulufteinlass 14 und gelangt in die Zuluftkammer 15. In der Zuluftkammer 15 wird die Zuluft beschleunigt und verliert dort an Druck. Dann durchströmt die Zuluft axial den ersten Schalldämper 17 und erreicht das Gebläse 18, dessen Lüfterrad dann drehangetrieben ist. Das Gebläse 18 drückt die Zuluft axial durch den zweiten Schalldämpfer 19. Die Zuluft wird dann in dem Strömungs-Umkehrelement 20 um 180° umgelenkt. Die Zuluft durchtritt dann gleichmäßig den Zuluftauslass 21 über dessen gesamte Länge. Dazu kann ein schräg gestelltes Lochblech eingesetzt werden. Die Zuluft strömt dabei nach Verlassen der Belüftungsanordnung 9 zumindest bereichsweise unten an der Deckenanordnung 4 entlang und umströmt dabei die Ionisierungsnadeln 7.

Durch das Einführen der Zuluft in den Innenraum 6 entsteht in dem Innenraum 6 ein Überdruck. Der Überdruck führt dazu, dass die verbrauchte Luft bzw. Abluft in dem Innenraum 6 durch die Belüftungsanordnung 9 automatisch wieder abgeführt wird. Die Abluft tritt über die Ablufteinlässe 24 beidseitig in die Ablufteinrichtung ein und strömt in einer Abluft-Hauptströmungsrichtung zu dem gemeinsamen, zentralen Abluftauslass, wo diese die Belüftungsanordnung 9 wieder verlässt. Dabei wird die Abluft in der Ablufteinrichtung mäanderartig durch die Abluft-Umlenkkörper 25 umgelenkt, die Abluft-Umlenkstellen bilden.

An der Deckenordnung 4 sind unten mehrere Deckenleuchten 26 zum Erhellen des Innenraums 6 angeordnet.

An/in der Deckenanordnung 4 ist eine elektrische, insbesondere elektronische, Betätigungseinrichtung 27 angeordnet. Die Betätigungseinrichtung 27 steht mit den Deckenleuchten 26 zumindest zeitweise in elektrisch leitender Verbindung bzw. in Signalverbindung.

Die Betätigungseinrichtung 27 steht außerdem mit einem (Hochspannungs)Transformator 28 in elektrisch leitender Verbindung, der eine elektrische Eingangsspannung in eine elektrische Ausgangsspannung wandelt.

Der Transformator 28 steht ausgangsseitig mit dem Gebläse 18 in elektrisch leitender Verbindung bzw. Signalverbindung. Ferner steht der Transformator 28 ausgangsseitig mit jeder Platine der jeweiligen Ionisierungseinrichtung 2 in elektrisch leitender Verbindung bzw. in Signalverbindung.

Die Betätigungseinrichtung 27 ist drahtlos mittels einer Fernbedienung 29 manuell bedienbar. Sie hat dafür eine entsprechende Empfangseinheit.

Ein Einschalten der Deckenleuchten 26 mittels der Fernbedienung 29 führt gemäß einer beispielhaften Ausführungsform zu einem Einschalten der Ionisierungseinrichtungen 2. Die Betätigungseinrichtung 27 empfängt ein entsprechendes Betätigungssignal und schaltet die Deckenleuchten 26 und die Ionisierungseinrichtungen 2 ein. Die Deckenleuchten 26 und die Ionisierungseinrichtungen 2 werden dann mit elektrischer Energie versorgt. Es ist aber bevorzugt, wenn die Deckenleuchten 26 und die Ionisierungseinrichtungen 2 unabhängig voneinander bzw. separat ausgeführt sind. Ein Einschalten und Ausschalten der Deckenleuchten 26 führt bevorzugt zu keiner Betätigung der Ionisierungseinrichtungen 2.

Ein Einschalten der Belüftungsanordnung 9 mittels der Fernbedienung 29 bewirkt gemäß einer bevorzugten Ausführungsform ein Einschalten der Ionisierungseinrichtungen 2. Die Betätigungseinrichtung 27 empfängt ein entsprechendes Betätigungssignal und schaltet die Belüftungsanordnung 9 und die Ionisierungseinrichtungen 2 ein. Die Belüftungsanordnung 9 und die Ionisierungseinrichtungen 2 werden dann mit elektrischer Energie versorgt.

Ein Ausschalten der Belüftungsanordnung 9 hat bevorzugt auch Einfluss auf den Betrieb der Ionisierungseinrichtungen 2. Sie werden dann ausgeschaltet. Die Ionisierungseinrichtungen 2 und die Belüftungsanordnung 9 sind, insbesondere funktional, miteinander gekoppelt.

Sofern vorhanden, erfasst eine, insbesondere deckenseitig angeordnete, Personen-Erfassungseinrichtung, ob sich in dem Innenraum 6 mindestens eine Person aufhält. Falls nein, werden die Ionisierungseinrichtungen 2 mit reduzierter Leistung oder bevorzugt alternativ gar nicht betrieben. Falls ja, werden die Ionisierungseinrichtungen 2 mit normaler oder erhöhter Leistung betrieben.

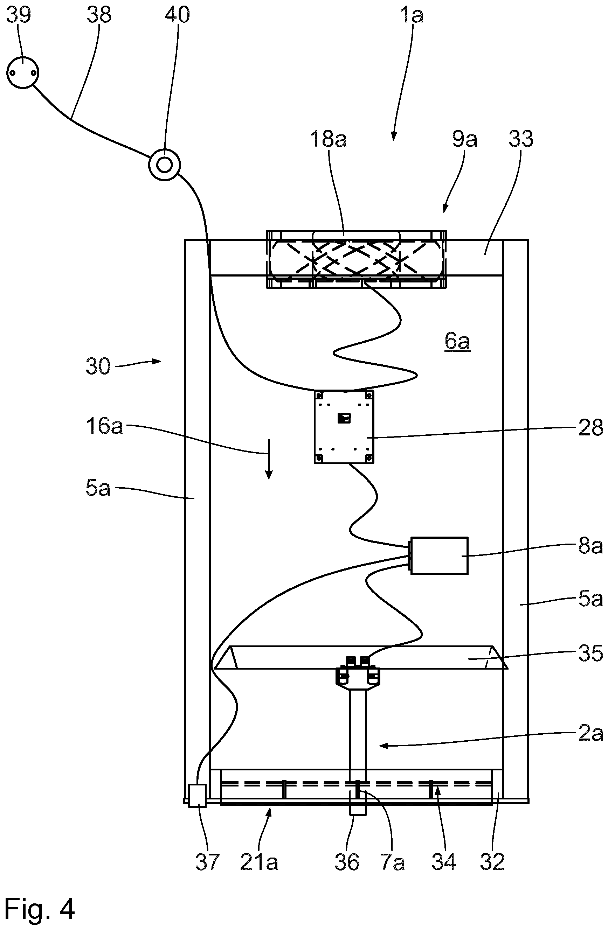

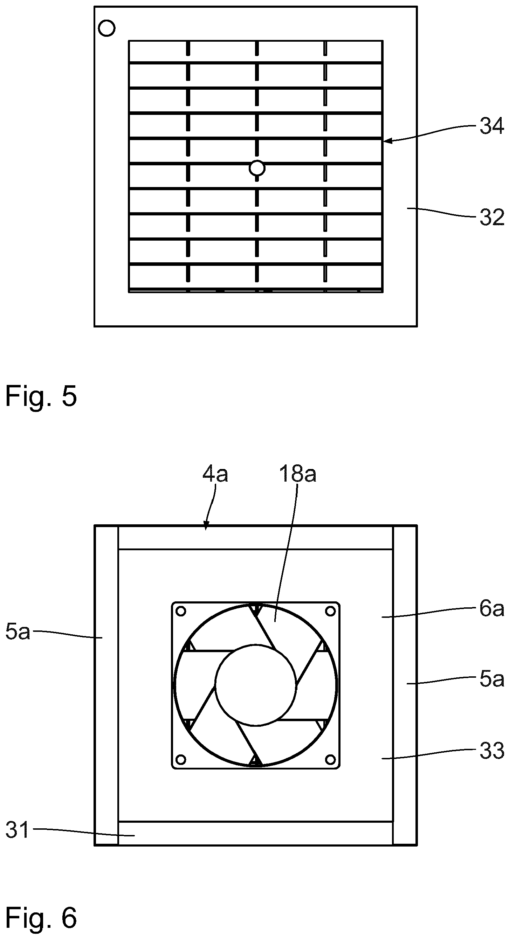

Nachfolgend wird unter Bezugnahme auf die Figuren 4 bis 6 eine alternative Ausführungsform einer Einrichtungsanordnung beschrieben. Konstruktiv identische Teile erhalten dieselben Bezugszeichen wie bei der vorherigen Ausführungsform, auf deren Beschreibung hiermit explizit verwiesen wird. Konstruktiv unterschiedliche, jedoch funktionell gleichartige Teile erhalten dieselben Bezugszeichen mit einem nachgeordneten "a".

Das eine Einrichtungsanordnung bildende Möbelstück 1a umfasst einen quaderförmigen Korpus 30, der wiederum eine Deckenanordnung 4a, Seitenwände 5a und einen Boden 31 aufweist. Ferner hat der Korpus 30 ein Vorderteil 32 und ein Rückteil 33. Die Deckenanordnung 4a und der Boden 31 verlaufen parallel und beabstandet zueinander. Sie erstrecken sich horizontal. Das Vorderteil 32 und das Rückteil 33 verlaufen parallel, aber beabstandet zueinander. Sie erstrecken sich wie die Seitenwände 5a vertikal. Durch die Deckenanordnung 4a, die Seitenwände 5a, den Boden 31 und das Vorderteil 32 sowie das Rückteil 33 ist ein Innenraum 6a des Möbelstücks 1a nach außen räumlich begrenzt.

Das Vorderteil 32 umfasst ein Lamellengitter 34 mit Luftauslasslamellen, die einen Luftauslass 21a ausbilden.

Das Rückteil 33 trägt ein Gebläse 18a, das Bestandteil der Luftstrom-Anordnung 9a ist. Das Gebläse 18a ist imstande, Luft von außen, wie von einem Raum, in den Innenraum 6a des Möbelstücks 1a zu führen. Die Luft kann über das Lamellengitter 34 den Innenraum 6a wieder verlassen. Das Gebläse 18a ist so imstande, im Betrieb einen horizontalen Luftstrom in dem Möbelstück 1a bzw. durch dieses zu erzeugen.

An dem Korpus 30 ist innenseitig eine Halterung 35 angeordnet. Die Halterung 35 erstreckt sich bevorzugt benachbart zu dem Lamellengitter 34 und bildet ein Tragelement für eine Ionisierungseinrichtung 2a. Die Halterung 35 ist von dem erzeugten Luftstrom umströmbar.

Die Ionisierungseinrichtung 2a ist an der Halterung 35 befestigt und erstreckt sich horizontal in der Luft-Hauptströmungsrichtung 16a des Luftstroms in dem Innenraum 6a. Sie weist eine Ionisierungsnadel 7 auf, die kopfseitig von einer Abdeckung 36 umgeben und von dem Luftstrom umströmbar ist. Die Ionisierungsnadel 7 ist ausgangsseitig in Bezug auf den Luftstrom an dem Korpus 30 und bevorzugt zentral bzw. mittig in Bezug auf einen Querschnitt des Möbelstücks 1a angeordnet. Sie ist benachbart zu dem Lamellengitter 34 und beispielsweise stromabwärts zu diesem in Bezug auf die Luft-Hauptströmungsrichtung 16a angeordnet.

Das Möbelstück 1a umfasst außerdem wieder eine Schutzbox 8a, die mit der Ionisierungseinrichtung 2a für deren Betrieb in elektrisch leitender Verbindung steht und in dem Korpus 30 angeordnet ist. Die Schutzbox 8a steht außerdem mit einer Betriebszustandsanzeige 37 in elektrischer Verbindung, die an dem Vorderteil 32 bzw. benachbart zu diesem an einer Seitenwand 5a, der Deckenanordnung 4a oder dem Boden 31 angeordnet ist.

Die Schutzbox 8a steht ferner mit dem Transformator 28 in elektrisch leitender Verbindung, der in dem Korpus 30 angeordnet ist und imstande ist, die Schutzbox 8a mit elektrischer Energie zu versorgen. Der Transformator 28 steht außerdem mit dem Gebläse 18a in elektrischer Verbindung, um dieses mit elektrischer Energie zu versorgen.

An dem Transformator 28 ist eingangsseitig ein Netzkabel 38 angeschlossen, das einen Netzstecker 39 zum Einstecken in eine Steckdose zur Energieversorgung umfasst.

In das Netzkabel 38 ist ein Betätigungsschalter 40 eingesetzt, der manuell betätigbar ist.

Die Ionisierungseinrichtung 2a und/oder das Gebläse 18a ist/sind durch Betätigung des Betätigungsschalters 40 betätigbar, der eine Ein-Stellung und eine Aus-Stellung hat. Wenn der Betätigungsschalter 40 in seine Ein-Stellung gebracht wird, werden die Ionisierungseinrichtung 2a und das Gebläse 18a eingeschaltet. Wenn der Betätigungsschalter 40 in seine Aus-Stellung gebracht wird, werden die Ionisierungseinrichtung 2a und das Gebläse 18a ausgeschaltet.

Beispielsweise hat das Möbelstück 1a auch eine Personen-Erfassungseinrichtung.

Bezüglich des genauen Aufbaus einzelner Komponenten des Möbelstücks 1a und der Ionisierung wird auf die vorherige Beschreibung verwiesen.

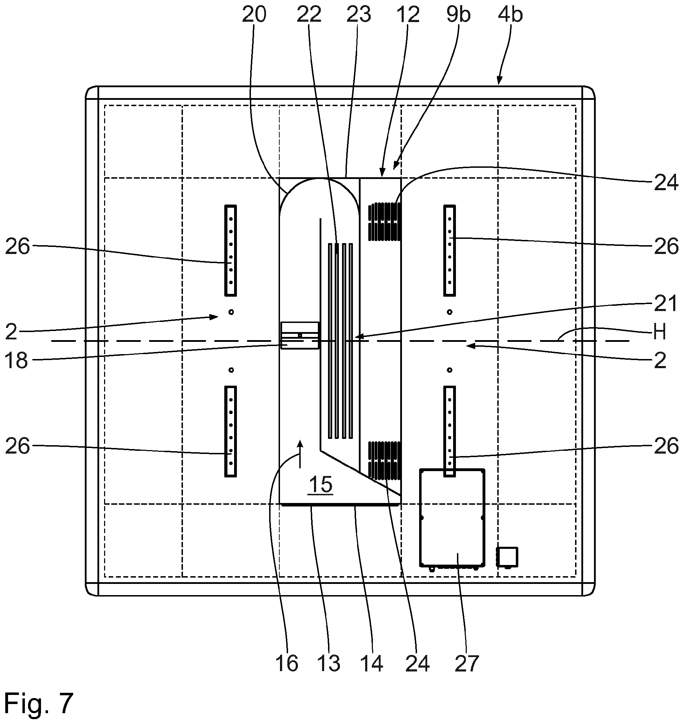

Nachfolgend wird unter Bezugnahme auf Fig. 7 eine alternative Ausführungsform einer Deckenanordnung beschrieben. Konstruktiv identische Teile erhalten dieselben Bezugszeichen wie bei der ersten Ausführungsform, auf deren Beschreibung hiermit explizit verwiesen wird. Konstruktiv unterschiedliche, jedoch funktionell gleichartige Teile erhalten dieselben Bezugszeichen mit einem nachgeordneten "b".

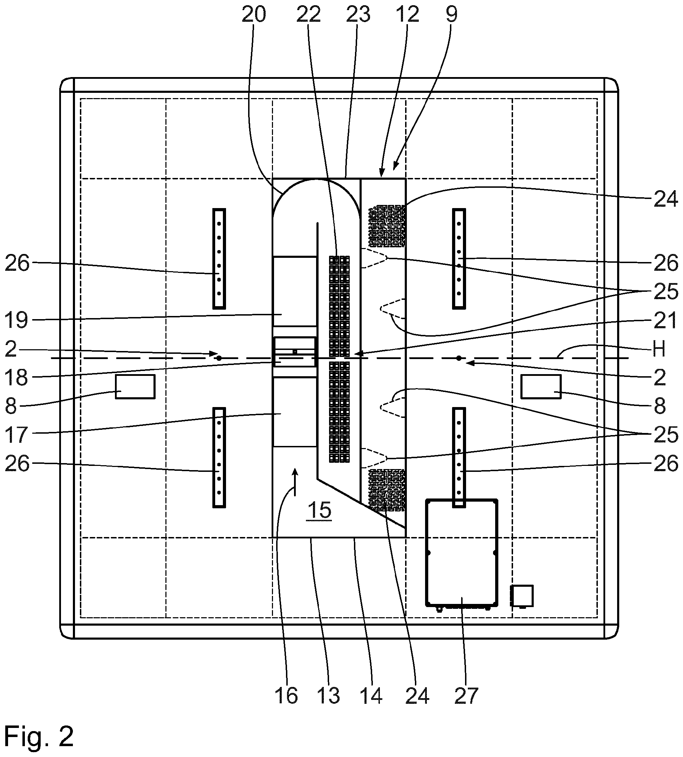

Im Vergleich mit der Deckenanordnung 4 gemäß Fig. 1, 2 sind bei der Deckenanordnung 4b gemäß Fig. 7 zwischen zwei Deckenleuchten 26 an zwei Seiten der zentralen Belüftungsanordnung 9b zwei Ionisierungseinrichtungen 2 bzw. Ionisierungsnadeln 7 beabstandet zueinander angeordnet. Die Belüftungsanordnung 9b ist somit zwischen den zwei Gruppierungen aus den zwei Deckenleuchten 26 und den zwei Ionisierungseinrichtungen 2 bzw. Ionisierungsnadeln 7 angeordnet. Die Deckenleuchten 26 und Ionisierungseinrichtungen 2 bzw. Ionisierungsnadeln 7 sind dezentral an der Deckenanordnung 4b und beabstandet zueinander angeordnet und beabstandet zu der Belüftungsanordnung 9b angeordnet. Die Deckenleuchten 26 sind jeweils lang/länglich. Jede Ionisierungseinrichtung 2 bzw. Ionisierungsnadel 7 hat bevorzugt zu der benachbarten Deckenleuchte 26 einen horizontalen Abstand, der zwischen 50 mm und 150 mm, bevorzugt zwischen 75 mm und 125 mm, bevorzugt bei 100 mm, liegt. Die auf einer Seite der Belüftungsanordnung 9b angeordneten Deckenleuchten 26 und Ionisierungseinrichtungen 2 bzw. Ionisierungsnadeln 7 sind jeweils (im Wesentlichen) in einer Reihe angeordnet. Bei der Ausführungsform gemäß Fig. 2 ist zwischen zwei Deckenleuchten 26 an zwei einander gegenüberliegenden Seiten der Belüftungsanordnung 9 mittig jeweils nur genau eine Ionisierungseinrichtung 2 bzw. Ionisierungsnadel 7 angeordnet.

Beispielsweise ist an der Deckenanordnung 4b auch eine Personen-Erfassungseinrichtung angeordnet.

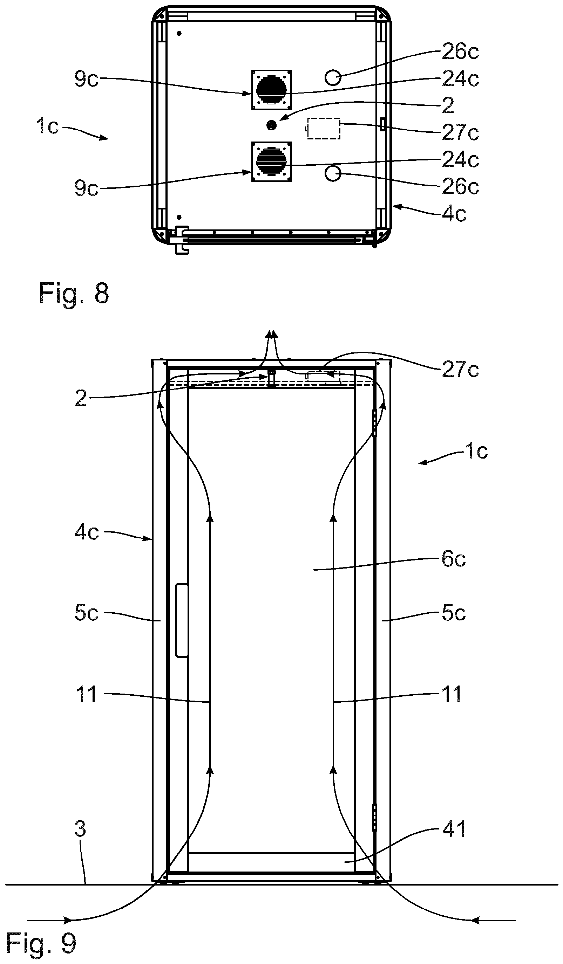

Nachfolgend wird unter Bezugnahme auf die Fig. 8, 9 eine alternative Ausführungsform einer Einrichtungsanordnung beschrieben. Konstruktiv identische Teile erhalten dieselben Bezugszeichen, insbesondere wie bei der vorherigen bzw. ersten Ausführungsform, auf deren Beschreibung hiermit explizit verwiesen wird. Konstruktiv unterschiedliche, jedoch funktionell gleichartige Teile erhalten dieselben Bezugszeichen mit einem nachgeordneten "c".

Das Raummodul 1c ist insbesondere ausgebildet, um in diesem Telefonate zu führen. Es hat eine quadratische Deckenanordnung 4c und eine abgehängte Decke, insbesondere aus Spanplatten, insbesondere melaminbeschichteten Spanplatten.

Im Zentrum der Deckenanordnung 4c ist genau eine Ionisierungseinrichtung 2 bzw. Ionisierungsnadel 7 angeordnet. Die Ionisierungseinrichtung 2 bzw. Ionisierungsnadel 7 befindet sich mittig zwischen zwei Belüftungsanordnungen 9c bzw. deren Ablufteinlässen 24c, die eine quadratische Grundform aufweisen und dezentral an der Deckenanordnung 4c angeordnet sind. Die Belüftungsanordnungen 9c bzw. deren Ablufteinlässe 24c und die Ionisierungseinrichtung 2 sind in einer gemeinsamen vertikalen Hauptebene der Deckenanordnung 4c und beabstandet zueinander angeordnet.

Seitlich neben der Ionisierungseinrichtung 2 bzw. Ionisierungsnadel 7 und beabstandet zu dieser ist an der Deckenanordnung 4c eine Betätigungseinrichtung 27c angeordnet. Die Betätigungseinrichtung 27c arbeitet ohne Steuerung.

Seitlich neben den Belüftungsanordnungen 9c bzw. den Ablufteinlässen 24c und beabstandet zu diesen sind an der Deckenanordnung 4c zwei Deckenleuchten 26c angeordnet. Die Deckenleuchten 26c haben einen runden Querschnitt bzw. eine runde Grundform.

Beispielsweise ist an der Deckenanordnung 4c auch eine Personen-Erfassungseinrichtung angeordnet.

Im Betrieb saugen die Belüftungsanordnungen 9c Luft aus dem Innenraum 6c ein. Die Luft gelangt über die Ablufteinlässe 24c und/oder mindestens einen entsprechenden Strömungskanal in mindestens einer Seitenwand 5c in die Deckenanordnung 4c. Luft aus der Umgebung gelangt dabei über eine Bodenanordnung 41 des Raummoduls 1c und/oder mindestens einen entsprechenden Strömungskanal in mindestens einer Seitenwand 5c in den Innenraum 6c. Eine kontinuierliche Luftumwälzung ist so wieder möglich. In dem Innenraum 6 strömt die Luft im Wesentlichen nach oben. Die Ionisierungsnadel 7 wird dabei umströmt. Sie ist einer Strömungs-Erzeugungsvorrichtung in Bezug auf die Strömung der Luft vorgeordnet.

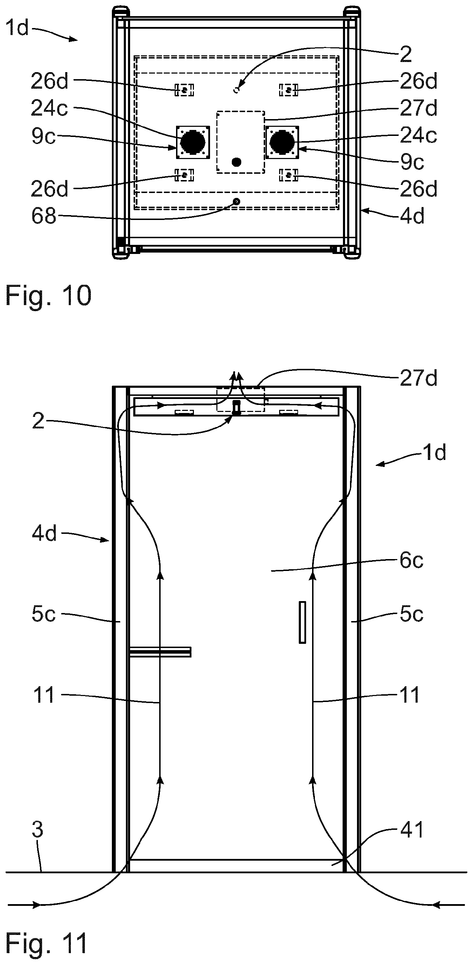

Nachfolgend wird unter Bezugnahme auf die Fig. 10, 11 eine alternative Ausführungsform einer Einrichtungsanordnung beschrieben. Konstruktiv identische Teile erhalten dieselben Bezugszeichen, insbesondere wie bei der vorherigen bzw. ersten Ausführungsform, auf deren Beschreibung hiermit explizit verwiesen wird. Konstruktiv unterschiedliche, jedoch funktionell gleichartige Teile erhalten dieselben Bezugszeichen mit einem nachgeordneten "d".

Die Einrichtungsanordnung gemäß Fig. 10, 11 unterscheidet sich von der Einrichtungsanordnung gemäß Fig. 8, 9 im Wesentlichen durch die Deckenanordnung 4d.

In einem zentralen Bereich der Deckenanordnung 4d ist die Betätigungseinrichtung 27d angeordnet. Die Betätigungseinrichtung 27d ist zwischen zwei dezentralen Belüftungsanordnungen 9c bzw. deren Ablufteinlässen 24c angeordnet.

Die Deckenanordnung 4d trägt außerdem vier Deckenleuchten 26d, die um die Betätigungseinrichtung 27d angeordnet sind. Die Deckenleuchten 26d sind in Eckbereichen eines imaginären Vierecks angeordnet und kürzer als bei der Ausführungsform gemäß Fig. 7.

Zwischen zwei Deckenleuchten 26d ist genau eine Ionisierungseinrichtung 2 bzw. Ionisierungsnadel 7 an der Deckenanordnung 4d angeordnet.

Zwischen zwei anderen Deckenleuchten 26d ist eine erste Belüftungsanordnung 9c bzw. ein erster Ablufteinlass 24c angeordnet. Zwischen zwei anderen Deckenleuchten 26d ist eine zweite Belüftungsanordnung 9c bzw. ein zweiter Ablufteinlass 24c an der Deckenanordnung 4d angeordnet.

Die Betätigungseinrichtung 27d ist zwischen den Belüftungsanordnungen 9c bzw. Ablufteinlässen 24c angeordnet. Die Ionisierungseinrichtung 2 bzw. Ionisierungsnadel 7 ist außermittig in Bezug auf die Deckenanordnung 4d angeordnet. Sie ist beabstandet zu der Betätigungseinrichtung 27d, den Deckenleuchten 26 und den Belüftungsanordnungen 9c bzw. deren Ablufteinlässen 24c angeordnet.

Eine Personen-Erfassungseinrichtung 68 ist an der Deckenanordnung 4d, günstigerweise randseititg bzw. dezentral, angeordnet. Die Betätigungseinrichtung 27d ist zwischen der Ionisierungseinrichtung 2 beziehungsweise Ionisierungsnadel 7 und der Personen-Erfassungseinrichtung 68 angeordnet. Die Personen-Erfassungseinrichtung 68, Ionisierungseinrichtung 2 beziehungsweise Ionisierungsnadel 7 und Betätigungseinrichtung 27d liegen (im Wesentlichen) in einer gemeinsamen vertikalen Hauptebene der Deckenanordnung 4d.

Der Betrieb des Raummoduls 1d ist ähnlich wie der Betrieb des Raummoduls 1c gemäß Fig. 8, 9, worauf verwiesen wird. Im Gegensatz zu der Ausführungsform gemäß Fig. 8, 9 steuert die Personen-Erfassungseinrichtung 68 bei Erfassung von mindestens einer Person, bevorzugt mit einer Nachlaufzeit, Licht und Belüftung an. Die Ionisierungseinrichtung 2 beziehungsweise Ionisierungsnadel 7 wird wie das Licht und die Belüftung aktiviert oder deaktiviert.

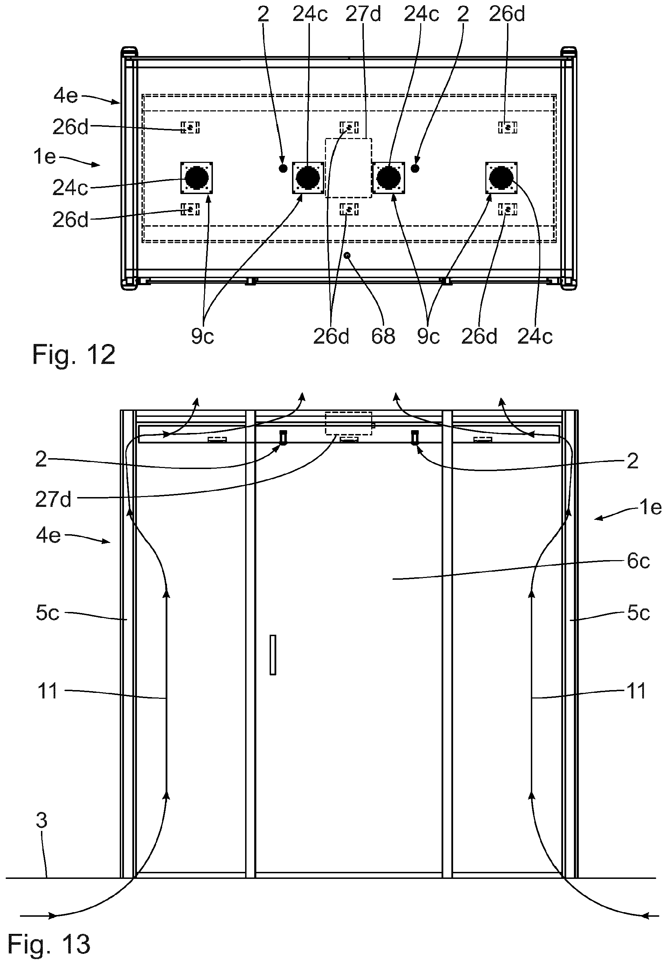

Nachfolgend wird unter Bezugnahme auf die Fig. 12, 13 eine alternative Ausführungsform einer Einrichtungsanordnung beschrieben. Konstruktiv identische Teile erhalten dieselben Bezugszeichen, insbesondere wie bei der vorherigen bzw. ersten Ausführungsform, auf deren Beschreibung hiermit explizit verwiesen wird. Konstruktiv unterschiedliche, jedoch funktionell gleichartige Teile erhalten dieselben Bezugszeichen mit einem nachgeordneten "e".

Die Einrichtungsanordnung gemäß Fig. 12, 13 ist v.a. für Besprechungen, Gespräche oder dergleichen ausgeführt. Sie hat eine längliche, rechteckige Grundform.

Die Einrichtungsanordnung gemäß Fig. 12, 13 unterscheidet sich v.a. in ihrer Deckenanordnung 4e von den vorherigen Ausführungsformen. In einem zentralen Bereich der Deckenanordnung 4e ist eine Betätigungseinrichtung 27d angeordnet.

An zwei einander gegenüberliegende Seiten der Betätigungseinrichtung 27d schließen sich zwei Belüftungsanordnungen 9c bzw. Ablufteinlässe 24c an. Alternativ sind zwei Belüftungsanordnungen 9c bzw. Ablufteinlässe 24c beabstandet, aber benachbart zu zwei einander gegenüberliegenden Seiten der Betätigungseinrichtung 27d angeordnet. Ferner sind zwei weitere Belüftungsanordnungen 9c bzw. Ablufteinlässe 24c in zwei einander gegenüberliegenden kurzen Randbereichen der Deckenanordnung 4e angeordnet. Die Belüftungsanordnungen 9c bzw. Ablufteinlässe 24c sind allesamt in einer vertikalen Hauptebene der Deckenanordnung 4e bzw. geringfügig versetzt gegenüber dieser und beabstandet zueinander angeordnet. Die Hauptebene erstreckt sich in einer Längsrichtung der Deckenanordnung 4e.

Ferner trägt die Deckenanordnung 4e insgesamt sechs Deckenleuchten 26d, die in zwei parallel zueinander verlaufenden Reihen beabstandet zueinander angeordnet sind. Diese Reihen erstrecken sich parallel zu der zuletzt erwähnten Hauptebene.

Die Deckenanordnung 4e trägt außerdem zwei Ionisierungseinrichtungen 2 bzw. Ionisierungsnadeln 7 auf, die ebenfalls in der vertikalen Hauptebene oder benachbart zu dieser angeordnet sind. Die Ionisierungseinrichtungen 2 bzw. Ionisierungsnadeln 7 sind benachbart, aber beabstandet zu den Belüftungsanordnungen 9c bzw. Ablufteinlässen 24c angeordnet, die benachbart zu der Betätigungseinrichtung 27d angeordnet sind bzw. an diese angrenzen. Sie sind beabstandet zu den äußeren Belüftungsanordnungen 9c bzw. Ablufteinlässen 24c und den Deckenleuchten 26d sowie der Betätigungseinrichtung 27d angeordnet.

Eine Personen-Erfassungseinrichtung 68 ist an der Deckenanordnung 4e, günstigerweise randseitig bzw. dezentral, angeordnet. Eine Deckenleuchte 26d ist zwischen der Betätigungseinrichtung 27d und der Personen-Erfassungseinrichtung 68 angeordnet. Die Betätigungseinrichtung 27d und die beiden inneren beziehungsweise mittleren Deckenleuchten 26d sowie die Personen-Erfassungseinrichtung 68 liegen (im Wesentlichen) in einer gemeinsamen vertikalen Hauptebene der Deckenanordnung 4e, die sich senkrecht zu der Längsrichtung der Deckenanordnung 4e erstreckt.

Der Betrieb ist wie bei der Ausführungsform gemäß Fig. 10, 11, worauf verwiesen wird.

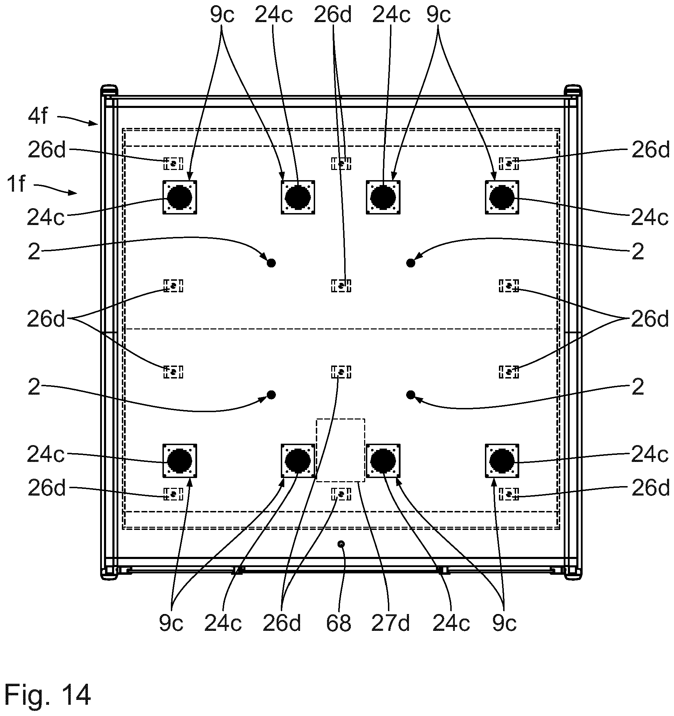

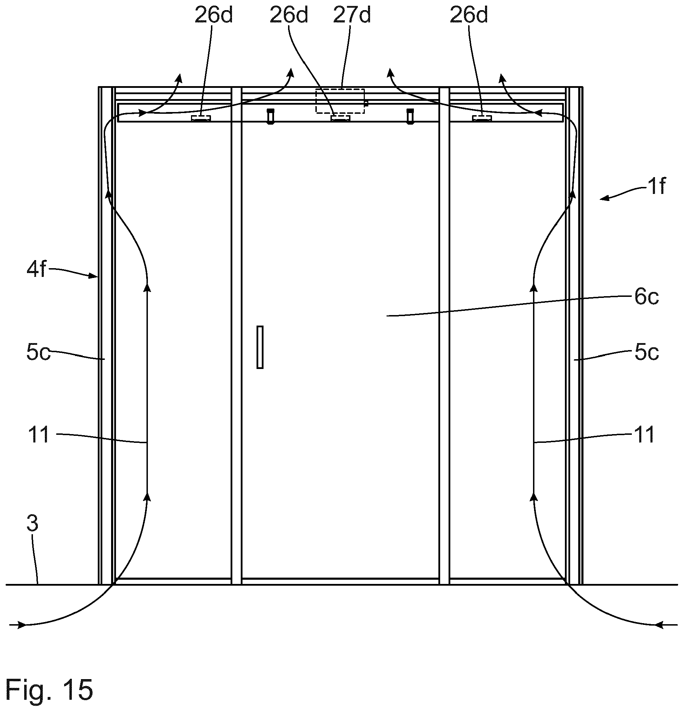

Nachfolgend wird unter Bezugnahme auf die Fig. 14, 15 eine alternative Ausführungsform einer Einrichtungsanordnung beschrieben. Konstruktiv identische Teile erhalten dieselben Bezugszeichen, insbesondere wie bei der vorherigen bzw. ersten Ausführungsform, auf deren Beschreibung hiermit explizit verwiesen wird. Konstruktiv unterschiedliche, jedoch funktionell gleichartige Teile erhalten dieselben Bezugszeichen mit einem nachgeordneten "f".

Diese Einrichtungsanordnung hat eine quadratische Grundform und ist beispielsweise für eine Zusammenarbeit konzipiert.

In der Deckenanordnung 4f sind insgesamt acht Belüftungsanordnungen 9c bzw. Ablufteinlässe 24c angeordnet, die in zwei Reihen zu je vier Belüftungsanordnungen 9c bzw. Ablufteinlässen 24c beabstandet zueinander und dezentral angeordnet sind.

Ferner sind an der Deckenanordnung 4f in einem zentralen Bereich vier Ionisierungseinrichtungen 2 bzw. Ionisierungsnadeln 7 angeordnet, die in Eckbereichen eines imaginären Quadrats beabstandet zueinander angeordnet sind. Die Ionisierungseinrichtungen 2 bzw. Ionisierungsnadeln 7 sind so zwischen den beiden Reihen der Belüftungsanordnungen 9c bzw. Ablufteinlässe 24c angeordnet.

Ferner ist eine Betätigungseinrichtung 27d zwischen zwei inneren Belüftungsanordnungen 9c bzw. Ablufteinlässen 24c angeordnet, die sich an diese seitlich anschließen bzw. benachbart zu dieser angeordnet sind. Die Betätigungseinrichtung 27d ist in einer vertikalen Hauptebene der Deckenanordnung 4f angeordnet.

Insgesamt sind außerdem zwölf Deckenleuchten 26d vorhanden, die in insgesamt vier Reihen angeordnet sind. Die Reihen erstrecken sich parallel zu den Reihen der Belüftungsanordnungen 9c bzw. Ablufteinlässe 24c. Jede Reihe von Belüftungsanordnungen 9c bzw. Ablufteinlässen 24c ist zwischen zwei benachbarten Reihen von Deckenleuchten 26d angeordnet. Zwei Deckenleuchten 26d befinden sich in dem imaginären Quadrat, das durch die Anordnung der Ionisierungseinrichtungen 2 bzw. Ionisierungsnadeln 7 beschrieben wird.

Die Ionisierungseinrichtungen 2 bzw. Ionisierungsnadeln 7 sind beabstandet zu den Belüftungsanordnungen 9c bzw. Ablufteinlässen 24c und den Deckenleuchten 26d sowie der Betätigungseinrichtung 27d angeordnet.

Eine Personen-Erfassungseinrichtung 68 ist an der Deckenanordnung 4f, insbesondere randseitig bzw. dezentral, angeordnet. Eine Deckenleuchte 26d ist zwischen der Betätigungseinrichtung 27d und der Personen-Erfassungseinrichtung 68 angeordnet. Die Betätigungseinrichtung 27d und die Personen-Erfassungseinrichtung 68 sind in der vertikalen Hauptebene der Deckenanordnung 4f angeordnet.

Der Betrieb ist wie bei der Ausführungsform gemäß Fig. 10, 11, worauf verwiesen wird.

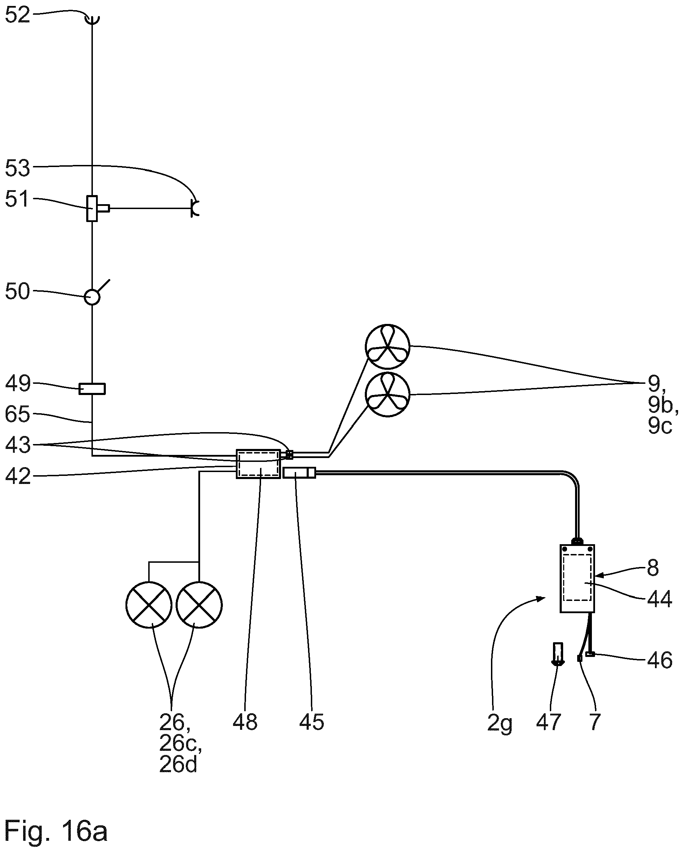

Nachfolgend wird unter Bezugnahme auf Fig. 16a ein vereinfachter Schaltplan beschrieben, der bei den Einrichtungsanordnungen alternativ Anwendung finden kann, insbesondere wenn keine Personen-Erfassungseinrichtung vorhanden ist.

An eine Verteilereinrichtung 42 ist die mindestens eine Deckenleuchte 26; 26c; 26d, beispielsweise über eine jeweilige Steckverbindung, anschließbar bzw. angeschlossen. Ferner ist an die Verteilereinrichtung 42 die mindestens eine Belüftungsanordnung 9; 9b; 9c, beispielsweise über eine jeweilige Steckverbindung 43, anschließbar bzw. angeschlossen. An die Verteilereinrichtung 42 ist außerdem mindestens eine Schutzbox 8, beispielsweise über eine jeweilige Steckverbindung 45, insbesondere in Form eines Wieland-Steckers, anschließbar bzw. angeschlossen. Jede Schutzbox 8 trägt eine Ionisierungsplatine 44.

Mit jeder Schutzbox 8 wiederum ist mindestens eine Ionisierungsnadel 7 elektrisch verbunden. Ferner ist mit jeder Schutzbox 8 ein Fuß 46 elektrisch verbunden, der mindestens ein Leuchtmittel für eine Betriebsanzeige der Ionisierung umfasst.

Jede Ionisierungsnadel 7 bzw. Ionisierungsnadel-Anordnung ist im Einsatz von einem im Wesentlichen hohlzylindrischen Grundkörper 47 aus PVC umgeben, die zusammen Bestandteil einer Ionisierungseinrichtung 2g sind. In montiertem Zustand sind der Fuß 46 und der Grundkörper 47 miteinander verbunden. Der Fuß 46 ist dann an der dem Innenraum 6 abgewandten Seite des Grundkörpers 47 angeordnet. Es ist zweckmäßig, wenn im Betrieb das mindestens eine Leuchtmittel durch den Grundkörper 47 scheint und für eine sich in dem Innenraum 6 aufhaltende Person sichtbar ist. Der Grundkörper 47 und der Fuß 46 sind Bestandteil eines Gehäuseteils 54 für die Ionisierungsnadel 7 bzw. Ionisierungsnadel-Anordnung. Jede Steckverbindung erlaubt eine einfache Verbindung mit der Verteilereinrichtung 42 bzw. eine einfache Trennung von dieser.

Die Verteilereinrichtung 42 umfasst ein Netzteil 48 zur Energieversorgung der mindestens einen Belüftungsanordnung 9; 9b; 9c, Deckenleuchte 26; 26c; 26d und der Ionisierungseinrichtung 2g.

An die Verteilereinrichtung 42 schließt sich außerdem eine elektrische Leitung 65 an, in die eine Steckverbindung 49 und ein Schalter 50 für die Betätigung der mindestens einen Deckenleuchte 26; 26c; 26d; Belüftungsanordnung 9; 9b; 9c und Ionisierungseinrichtung 2g sowie ein T-Verteiler 51 integriert sind.

Mit dem T-Verteiler 51 steht über eine elektrische Leitung ein Netzstecker 52 in elektrischer Verbindung. Ferner steht der T-Verteiler 51 in angeschlossenem Zustand über eine elektrische Leitung mit einer Steckdose 53 in elektrischer Verbindung.

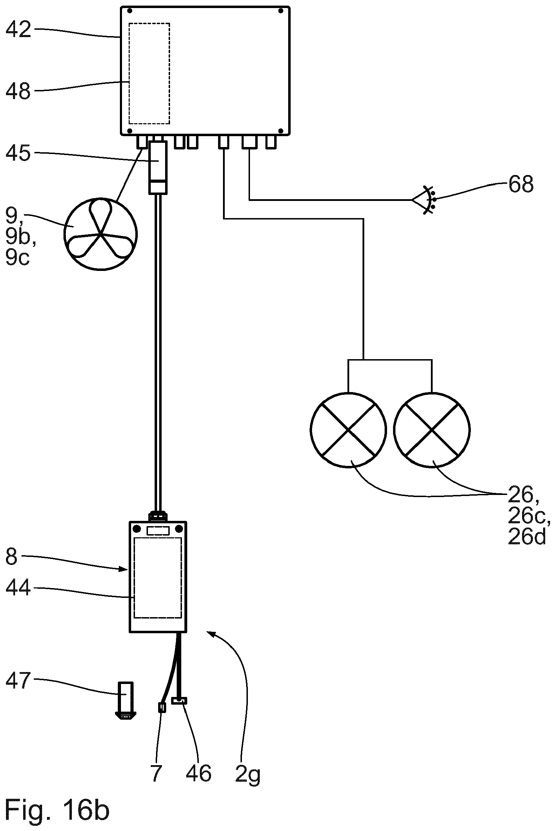

Nachfolgend wird unter Bezugnahme auf Fig. 16b ein vereinfachter Schaltplan beschrieben, der bei den Einrichtungsanordnungen alternativ Anwendung finden kann, insbesondere wenn eine Personen-Erfassungseinrichtung vorhanden ist.

An die Verteilereinrichtung 42 ist die mindestens eine Deckenleuchte 26; 26c; 26d, beispielsweise über eine jeweilige Steckverbindung, anschließbar beziehungsweise angeschlossen. Ferner ist an die Verteilereinrichtung 42 die mindestens eine Belüftungsanordnung 9; 9b; 9c, beispielsweise über eine jeweilige Steckverbindung, anschließbar beziehungsweise angeschlossen. An die Verteilereinrichtung 42 ist außerdem mindestens eine Schutzbox 8, beispielsweise über eine jeweilige Steckverbindung, insbesondere in Form eines Wieland-Steckers, anschließbar, beziehungsweise angeschlossen. An die Verteilereinrichtung 42 ist außerdem die Personen-Erfassungseinrichtung 68, beispielsweise über eine jeweilige Steckverbindung, anschließbar beziehungsweise angeschlossen.

Die Verteilereinrichtung 42 umfasst ein Netzteil 48 zur Energieversorgung der mindestens einen Belüftungsanordnung 9; 9b; 9c, Deckenleuchte 26; 26c; 26d und Ionisierungseinrichtung 2g sowie Personen-Erfassungseinrichtung 68. Das Netzteil 48 steht mit einem elektrischen Gebäudenetz oder einer anderen Energiequelle im Betrieb in elektrischer Verbindung. Die Personen-Erfassungseinrichtung 68 ist imstande, die Ionisierung, Beleuchtung und Belüftung einzuschalten, wenn mindestens eine Person erfasst wird.

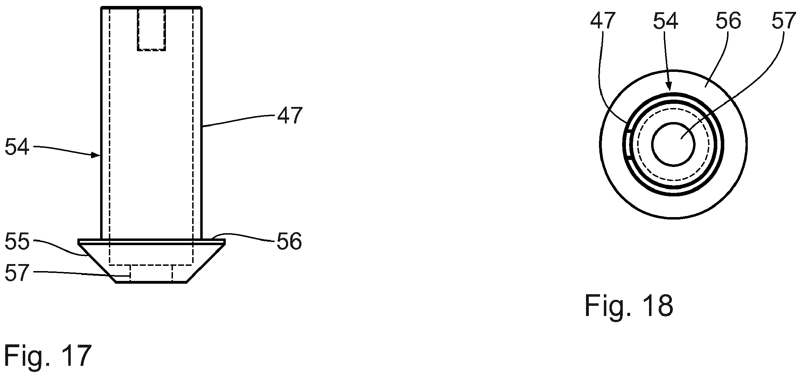

Nachfolgend Bezug nehmend auf die Fig. 17, 18 wird das Gehäuseteil 54 näher beschrieben. Es umfasst den Grundkörper 47 und einen kegelstumpfförmigen Kopf 55, der sich mit seiner Grundfläche 56 an den Grundkörper 47 anschließt und mit der Grundfläche 56 radial gegenüber dem Grundkörper 47 vorsteht.

In dem Kopf 55 ist zentral eine Ionisierungsöffnung 57 ausgebildet. Die Ionisierungsöffnung 57 erstreckt sich zwischen einer Deckfläche und der Grundfläche 54 des Kopfs 55. Im Einsatz ist der Kopf 55 dem Innenraum 6 zugewandt bzw. ragt er in diesen. Die Deckfläche ist in montiertem Zustand nach unten gewandt.

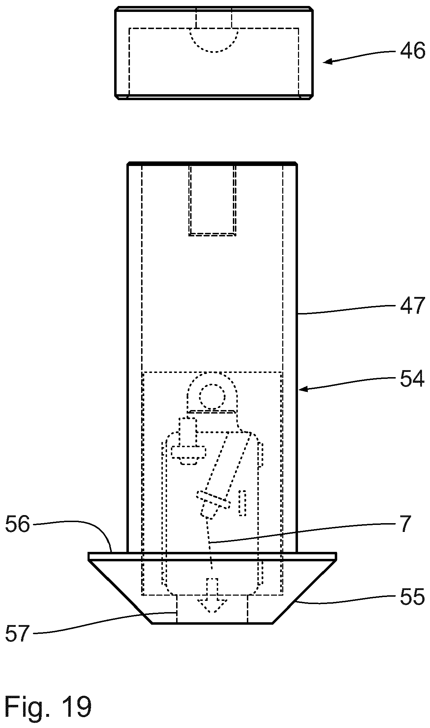

Wie Fig. 19 zeigt, ist in dem Gehäuseteil 54 die Ionisierungsnadel 7 bzw. Ionisierungsnadel-Anordnung untergebracht, die sich in die Ionisierungsöffnung 57 bzw. in Richtung auf diese erstreckt. Die Ionisierungsnadel 7 bzw. Ionisierungsnadel-Anordnung ist dort von einer Halteeinrichtung gehalten. Günstigerweise ist die Ionisierungsnadel 7 bzw. Ionisierungsnadel-Anordnung zwischen 2 mm und 6 mm, bevorzugt zwischen 3 mm und 5 mm, von der Deckfläche des Kopfes 55 aus Sicherheitsgründen rückversetzt.

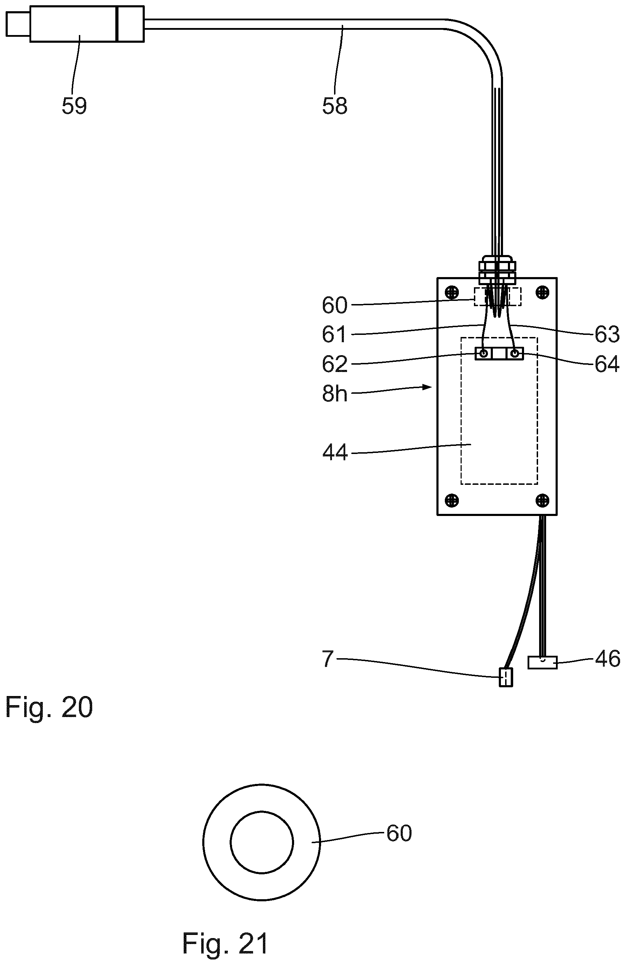

Nachfolgend wird unter Bezugnahme auf die Fig. 20, 21 eine alternative Schutzbox 8h näher beschrieben. Auf die vorherigen Ausführungen zu der Schutzbox 8 wird verwiesen.

In einem Anschlussbereich der Schutzbox 8h schließt sich an diese eine elektrische Leitung 58 an, die gegenüberliegend zu der Schutzbox 8h einen Stecker 59 trägt.

In der Schutzbox 8h ist benachbart zu dem Anschlussbereich ein Ferritring 60 angeordnet. Der Ferritring 60 ist zwischen dem Anschlussbereich und der Ionisierungsplatine 44 angeordnet.

Ein erster isolierter Leiter 61 der elektrischen Leitung 58 ist mit einem Masseanschluss 62 der Ionisierungsplatine 44 elektrisch verbunden. Ein zweiter isolierter Leiter 63 der Leitung 58 ist mit einem Netzanschluss 64 (12 V DC) der Ionisierungsplatine 44 elektrisch verbunden. Die Leiter 61, 63 sind um den Ferritring 60 gewickelt und durchsetzen jeweils drei Mal dessen zentrale Öffnung. Die Leiter 61, 63 und deren Windungen sind dabei nebeneinander angeordnet.

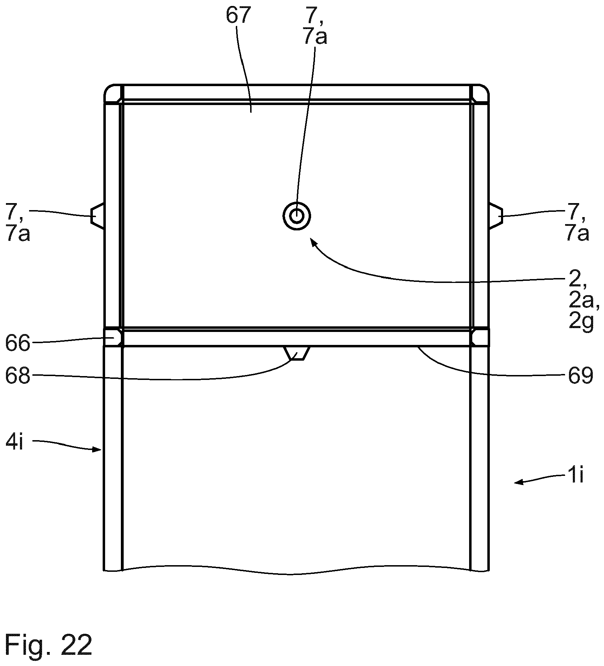

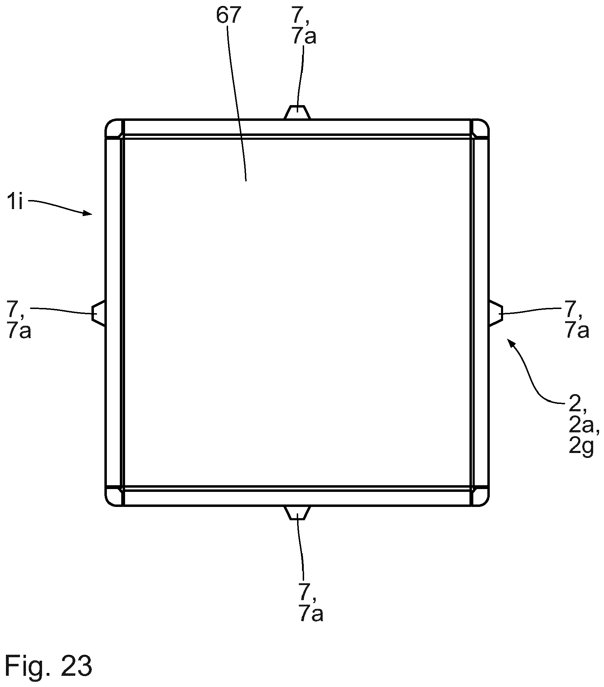

Nachfolgend wird unter Bezugnahme auf die Fig. 22, 23 eine alternative Ausführungsform einer Einrichtungsanordnung beschrieben. Konstruktiv identische Teile erhalten dieselben Bezugszeichen wie bei den vorherigen Ausführungsformen, auf deren Beschreibung hiermit explizit verwiesen wird. Konstruktiv unterschiedliche, jedoch funktionell gleichartige Teile erhalten dieselben Bezugszeichen mit einem nachgeordneten "i".

Das eine Einrichtungsanordnung bildende Möbelstück 1i ist beispielsweise als Theke, Rollcontainer, Schrank, Regal, Sideboard oder dergleichen ausgeführt. Es hat bevorzugt eine quaderförmige Grundform. Alternativ ist es aus mehreren quaderförmigen Grundkörpern gebildet. Andere Formen sind möglich.

Das Möbelstück 1i ist durch längliche, insbesondere rohrartige oder stangenartige, Tragelemente 4i gebildet, die in Knotenpunkten 66 miteinander verbunden sind. Das Möbelstück 1i hat horizontal verlaufende Tragelemente 4i und vertikal verlaufende Tragelemente 4i. Die verwendeten Tragelemente 4i können sich in ihrer Länge unterscheiden.

An mindestens einem Tragelement 4i ist gemäß einer bevorzugten Ausführungsform mindestens eine Tür, Klappe oder dergleichen, insbesondere schwenkbar, gelagert. Das Möbelstück 1i kann alternativ oder zusätzlich mindestens einen verschiebbar geführten Schub umfassen.

Das Möbelstück 1i trägt oben vier Ionisierungseinrichtungen 2; 2a; 2g, die gemäß einer der vorherigen Ausführungsformen ausgebildet sind. Die Ionisierungseinrichtungen 2; 2a; 2g befinden sich kopfseitig in dem Möbelstück 1i.

Jede Ionisierungseinrichtung 2i hat eine Ionisierungsnadel 7; 7a, die gemäß einer der vorherigen Ausführungsformen ausgebildet ist. Jede Ionisierungsnadel 7; 7a ist in einem zentralen Bereich eines vertikal verlaufenden Paneels 67 des Möbelstücks 1i angeordnet. Jedes Paneel 67 ist von mindestens einem Tragelement 4i, direkt oder indirekt, gehalten und ist bevorzugt rechteckförmig. Die Paneele 67 schließen sich seitlich aneinander an und begrenzen einen Innenraum zur Aufnahme der Ionisierungseinrichtungen 2i nach seitlich außen. Benachbarte Paneele 67 erstrecken sich senkrecht zueinander. Der Innenraum ist bevorzugt auch nach oben durch eine Decke und/oder unten durch einen Boden 69 begrenzt. Es ist zweckmäßig, wenn lediglich an Paneelen 67 eine Ionisierungsnadel 7; 7a angeordnet ist, die im Einsatz frei liegen bzw. beabstandet zu einer Wand, einem weiteren Möbelstück oder dergleichen verlaufen. Die Ionisierungsnadeln 7; 7a erstrecken sich zumindest bereichsweise durch das jeweilige Paneel 67 nach außen in den jeweiligen umgebenden Raum. Günstigerweise springen jeweils einander gegenüberliegende Ionisierungsnadeln 7; 7a von einem Grundkörper des Möbelstücks 1i voneinander weg. Es ist zweckmäßig, wenn die an benachbarten Paneelen 67 angeordneten Ionisierungsnadeln 7; 7a sich senkrecht zueinander erstrecken. Die Ionisierungsnadeln 7; 7a befinden sich bevorzugt in einer gemeinsamen horizontalen Ebene. Alternativ ist mindestens eine Ionisierungsnadel 7; 7a entsprechend an mindestens einem horizontal verlaufenden Paneel 67 angeordnet.

Das Möbelstück 1i umfasst außerdem eine Personen-Erfassungseinrichtung 68, die bevorzugt an dem Boden 69 außenseitig angeordnet ist. Günstigerweise ist die Personen-Erfassungseinrichtung 68 kopfseitig an dem Möbelstück 1i unterhalb der Ionisierungseinrichtungen 2; 2a; 2g angeordnet.

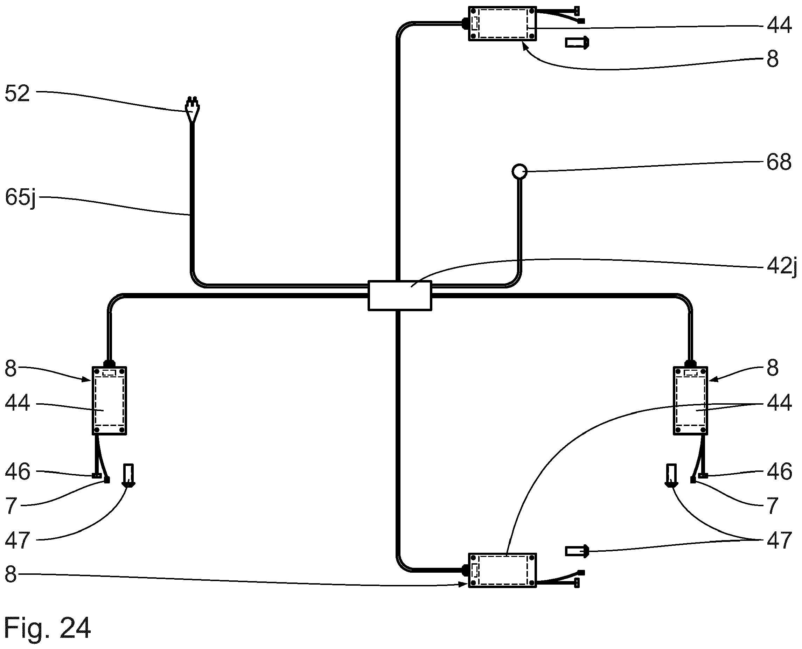

Nachfolgend wird unter Bezugnahme auf Fig. 24 ein vereinfachter Schaltplan beschrieben, der bei dem Möbelstück 1i gemäß Fig. 22, 23 Anwendung finden kann. Identische Teile erhalten dieselben Bezugszeichen wie bei dem Schaltplan gemäß Fig. 16. Konstruktiv unterschiedliche, jedoch funktionell gleichartige Teile erhalten dieselben Bezugszeichen mit einem nachgeordneten "j".

An die Verteilereinrichtung 42j sind vier Schutzboxen 8, beispielsweise über eine jeweilige Steckverbindung, anschließbar bzw. angeschlossen. Jede Schutzbox 8 trägt eine Ionisierungsplatine 44.

Mit jeder Schutzbox 8 wiederum ist mindestens eine Ionisierungsnadel 7; 7a elektrisch verbunden. Ferner ist mit jeder Schutzbox 8 ein Fuß 46 elektrisch verbunden, der mindestens ein Leuchtmittel für eine Betriebsanzeige der Ionisierung umfasst.

Jede Ionisierungsnadel 7; 7a ist im Einsatz von einem im Wesentlichen hohlzylindrischen Grundkörper 47 aus PVC umgeben, die zusammen Bestandteil einer Ionisierungseinrichtung 2; 2a; 2g sind.

Die Verteilereinrichtung 42j umfasst ein Netzteil zur Energieversorgung der Ionisierungseinrichtungen 2; 2a; 2g. An die Verteilereinrichtung 42j ist ein Netzkabel 65j mit einem Netzstecker 52 angeschlossen.

Ferner steht die Verteilereinrichtung 42j mit der Personen-Erfassungseinrichtung 68 zur Energieversorgung derselben in elektrischer Verbindung.

Wenn die Personen-Erfassungseinrichtung 68 mindestens eine Person erfasst, werden die Ionisierungseinrichtungen 2; 2a; 2g, bevorzugt für eine bestimmte Zeitdauer, eingeschaltet oder ausgeschaltet.

Kombinationen der einzelnen Ausführungsformen sind möglich.

## Patentansprüche

1. Einrichtungsanordnung, insbesondere für Gebäude,
a) mit mindestens einem Tragelement (4; 35; 4b; 4c; 4d; 4e; 4f; 4i, 67),
b) mit mindestens einer an dem mindestens einen Tragelement (4; 35; 4b; 4c; 4d; 4e; 4f; 4i, 67) angeordneten Ionisierungseinrichtung (2; 2a; 2g), die mindestens ein Ionisierungselement (7; 7a) zur Ionisierung gasförmiger Medien, insbesondere von Luft, aufweist, und
c) mit einer mit der mindestens einen Ionisierungseinrichtung (2; 2a; 2g) zumindest zeitweise in Verbindung stehenden Betätigungseinrichtung (27; 27c; 40) zum zumindest zeitweisen Betätigen der mindestens einen Ionisierungseinrichtung (2; 2a; 2g).

2. Einrichtungsanordnung nach Anspruch 1, **dadurch gekennzeichnet, dass** die Betätigungseinrichtung (27; 27c; 27d) eine Steuerung und/oder Regelung zum Betätigen des mindestens einen Ionisierungselements (7) aufweist.

3. Einrichtungsanordnung nach Anspruch 1 oder 2, **gekennzeichnet durch** mindestens eine Luftstrom-Anordnung (9; 9a; 9b; 9c) zum Erzeugen von mindestens einem Luftstrom.

4. Einrichtungsanordnung nach Anspruch 3, **dadurch gekennzeichnet, dass** das mindestens eine Ionisierungselement (7; 7a) derart an dem mindestens einen Tragelement (4; 35; 4b; 4c; 4d; 4e; 4f) angeordnet ist, dass der mindestens eine Luftstrom im Betrieb der mindestens einen Luftstrom-Anordnung (9; 9a; 9b; 9c) auf dieses trifft.

5. Einrichtungsanordnung nach Anspruch 3 oder 4, **dadurch gekennzeichnet, dass** die Betätigungseinrichtung (27; 27c; 27d; 40) mit der mindestens einen Luftstrom-Anordnung (9; 9a; 9b; 9c) zum zumindest zeitweisen Betätigen derselben zumindest zeitweise in Verbindung steht.

6. Einrichtungsanordnung nach Anspruch 5, **dadurch gekennzeichnet, dass** die mindestens eine Ionisierungseinrichtung (2; 2a; 2g) durch Betätigung der mindestens einen Luftstrom-Anordnung (9; 9a; 9b; 9c) betätigbar ist.

7. Einrichtungsanordnung nach einem der Ansprüche 3 bis 6, **dadurch gekennzeichnet, dass** die mindestens eine Luftstrom-Anordnung (9; 9a; 9b; 9c) in unterschiedlichen Stufen betreibbar ist und die mindestens eine Ionisierungseinrichtung (2; 2a; 2g) im Betrieb stets eine gleiche Ionisierungsleistung aufweist.

8. Einrichtungsanordnung nach einem der vorherigen Ansprüche, **gekennzeichnet durch** mindestens eine Beleuchtungsanordnung (26; 26c; 26d), die mit der Betätigungseinrichtung (27; 27c; 27d) zur zumindest zeitweisen Betätigung zumindest zeitweise in Verbindung steht, wobei die mindestens eine Ionisierungseinrichtung (2; 2g) durch Betätigung der mindestens einen Beleuchtungsanordnung (26; 26c; 26d) betätigbar ist.

9. Einrichtungsanordnung nach einem der vorherigen Ansprüche, **gekennzeichnet durch** mindestens eine Personen-Erfassungseinrichtung (68), wobei die mindestens einen Personen-Erfassungseinrichtung (68) mit der Betätigungseinrichtung (27; 27c; 27d) zumindest zeitweise in Verbindung steht und die Betätigungseinrichtung (27; 27c; 27d) die mindestens eine Ionisierungseinrichtung (2; 2g) bei mindestens einer erfassten Person anders als bei keiner erfassten Person betätigt.

10. Einrichtungsanordnung nach einem der vorherigen Ansprüche,
**dadurch gekennzeichnet, dass** das mindestens eine Tragelement (4; 4b; 4c; 4d; 4e; 4f) Bestandteil eines Raummoduls (1; 1c; 1d; 1e; 1f) ist, das vorzugsweise mindestens eine Seitenwand (5; 5c) und bevorzugt eine mit der mindestens einen Seitenwand (5, 5c) in Verbindung stehende Deckenanordnung zum räumlichen Begrenzen eines Raums (6) aufweist.

11. Einrichtungsanordnung nach einem der Ansprüche 1 bis 9, **dadurch gekennzeichnet, dass** das mindestens eine Tragelement (35; 4i) Bestandteil eines Möbelstücks, wie eines Tischs, insbesondere Schreibtischs, eines Regals, einer Kommode, eines Schranks, einer Garderobe, einer Theke, insbesondere Empfangstheke, eines Containers, insbesondere Rollcontainers, und/oder eines Sitzmöbelstücks, insbesondere eines (Büro-)Stuhls, einer Couch, einer Bank, eines Sessels oder dergleichen, ist.

12. Einrichtungsanordnung nach einem der vorherigen Ansprüche,
**dadurch gekennzeichnet, dass** das mindestens eine Ionisierungselement (7; 7a) in mindestens einem Gehäuseteil (54) zumindest teilweise untergebracht ist.

13. Einrichtungsanordnung nach Anspruch 12, **dadurch gekennzeichnet, dass** das mindestens eine Gehäuseteil (54) zumindest bereichsweise aus einem Polymermaterial, insbesondere thermoplastischem Polymermaterial, insbesondere PVC, gebildet ist.

14. Einrichtungsanordnung nach Anspruch 12 oder 13, **dadurch gekennzeichnet, dass** das mindestens eine Gehäuseteil (54) mindestens einen Kopf (55) aufweist, der sich zu seinem freien Ende hin zumindest bereichsweise, vorzugsweise kontinuierlich, verjüngt.

15. Einrichtungsanordnung nach einem der vorherigen Ansprüche,
**dadurch gekennzeichnet, dass** die mindestens eine Ionisierungseinrichtung (2; 2a; 2g) mindestens ein Schutzgehäuse (8h) umfasst, das mindestens eine Ionisierungsplatine (44) und mindestens einen dieser vorgeordneten Ferritkörper (60) zur Reduzierung von Störfrequenzen aufweist.

16. Einrichtungsanordnung nach Anspruch 15, **dadurch gekennzeichnet, dass** der mindestens eine Ferritkörper (60) mindestens eine Hindurchführöffnung aufweist, durch die mindestens ein Leiter (61, 63) einer elektrischen Leitung (58) für die mindestens eine Ionisierungsplatine (44), bevorzugt mehrfach, bevorzugt dreifach, geführt ist.
